(19) Europäisches Patentamt
European Patent Office
Office européen des brevets

(11) **EP 3 629 025 B1**

(12) **EUROPEAN PATENT SPECIFICATION**

(45) Date of publication and mention of the grant of the patent:
**21.04.2021 Bulletin 2021/16**

(51) Int Cl.:
***G01N 33/68*** *(2006.01)*

(21) Application number: **19198884.9**

(22) Date of filing: **23.09.2019**

# (54) METHOD FOR OBTAINING INFORMATION ON DIABETES AND USE THEREOF

VERFAHREN ZUR ERLANGUNG VON INFORMATIONEN ÜBER DIABETES UND VERWENDUNG DAVON

PROCÉDÉ PERMETTANT D'OBTENIR DES INFORMATIONS SUR LE DIABÈTE ET UTILISATION ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2018 JP 2018182399**

(43) Date of publication of application:
**01.04.2020 Bulletin 2020/14**

(73) Proprietors:
- **Kyushu University, National University Corporation Nishi-ku, Fukuoka-shi Fukuoka 819-0395 (JP)**
- **National University Corporation Kobe University Kobe-shi, Hyogo 657-8501 (JP)**
- **SYSMEX CORPORATION Kobe-shi Hyogo 651-0073 (JP)**

(72) Inventors:
- **Ninomiya, Toshiharu Fukuoka, 812-8581 (JP)**
- **Hirakawa, Yoichiro Fukuoka, 812-8581 (JP)**
- **Toh, Ryuji Hyogo, 657-8501 (JP)**
- **Irino, Yasuhiro Hyogo, 657-8501 (JP)**
- **Murakami, Katsuhiro Hyogo, 651-0073 (JP)**
- **Miwa, Keiko Hyogo, 651-0073 (JP)**
- **Harada, Amane Hyogo, 651-0073 (JP)**

(74) Representative: **Hoffmann Eitle Patent- und Rechtsanwälte PartmbB Arabellastraße 30 81925 München (DE)**

(56) References cited:
**US-A1- 2013 143 748 US-A1- 2016 109 469**
**US-A1- 2017 315 112**

- **Yoichiro Hirakawa: "2456-PUB: Cholesterol Uptake Capacity of High-Density Lipoprotein Predicts the Risk of Future Diabetes: The Hisayama Study | Diabetes", , 1 June 2019 (2019-06-01), XP055665085, Retrieved from the Internet: URL:https://diabetes.diabetesjournals.org/content/68/Supplement_1/2456-PUB [retrieved on 2020-02-04]**
- **AMANE HARADA ET AL: "Cholesterol Uptake Capacity: A New Measure of HDL Functionality for Coronary Risk Assessment", THE JOURNAL OF APPLIED LABORATORY MEDICINE: AN AACC PUBLICATION, vol. 2, no. 2, 31 May 2017 (2017-05-31), pages 186-200, XP055665095, DOI: 10.1373/jalm.2016.022913**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 629 025 B1

## Description

### TECHNICAL FIELD

The present invention relates to a method for obtaining information on diabetes. The present invention relates to a method for determining onset risk of diabetes. The present invention relates to a reagent kit for obtaining information on diabetes. The present invention relates to a device and computer program for determining onset risk of diabetes.

### BACKGROUND

Diabetes is a metabolic disease with symptoms of chronic hyperglycemia and glycosuria. Diabetes, in particular, type 2 diabetes, develops due to a decrease in insulin secretion and sensitivity due to genetic factors as well as environmental factors such as hyperphagia, obesity, and lack of exercise. The number of patients with diabetes is estimated to exceed 400 million worldwide as of 2017 and is also expected to increase in the future. In addition, diabetes is known to cause various complications such as cardiovascular disease, neuropathy, nephropathy, and retinopathy, and the problem is decrease in quality of life of the patient and increase in medical expenses. On the other hand, it is known that the diabetes onset risk can be reduced by diet and exercise therapy to improve lifestyle. From the viewpoint of public health and medical economy, it is important to find out a group of high diabetes onset risk and to actively carry out interventions to improve lifestyle.

For diagnosis of diabetes, fasting blood glucose, glucose tolerance test and measured values of hemoglobin A1c (HbA1c) are used. When these values are lower than values diagnosed with diabetes but higher than normal value, it is diagnosed with borderline type. The borderline type is known to have a higher diabetes onset risk than healthy person. In addition, Blanco-Rojo, R. et al., HDL cholesterol efflux normalized to apoA-I is associated with future development of type 2 diabetes: from the CORDIOPREV trial. Sci. Rep., 2017, 7(1): 12499 has examined association between cholesterol efflux from macrophages by high-density lipoprotein (HDL) and onset of type 2 diabetes. Blanco-Rojo, R. et al., HDL cholesterol efflux normalized to apoA-I is associated with future development of type 2 diabetes: from the CORDIOPREV trial. Sci. Rep., 2017, 7(1): 12499 has described that a value obtained by dividing cholesterol efflux rate by the amount of ApoAI protein (ratio of cholesterol efflux/ApoAI) is associated with the onset of type 2 diabetes. On the other hand, Blanco-Rojo, R. et al., HDL cholesterol efflux normalized to apoA-I is associated with future development of type 2 diabetes: from the CORDIOPREV trial. Sci. Rep., 2017, 7(1): 12499 has shown that the cholesterol efflux rate itself was not significantly different between a non-diabetic subject group and a diabetic subject group.

In US 2013/0143748 A1 a method of measuring the expression level of FSTL3 gene in a biological sample and correlating the measured expression level with the detection risk of developing diabetes is described.

An object of the present invention is to provide a new means capable of predicting diabetes onset risk.

### SUMMARY

The present inventors have found that a measured value of ability to uptake sterol per unit volume of a biological sample of a subject serves as an indicator of diabetes onset risk, and thus have completed the present invention. Thus, the present invention provides a method for obtaining information on subject's diabetes, including measuring lipoprotein's ability to uptake sterol in a biological sample of a subject, in which the measured value of ability to uptake sterol per unit volume of the biological sample is an indicator of diabetes onset risk.

The present invention provides a method for obtaining information on subject's diabetes, comprising measuring lipoprotein's ability to uptake sterol in a biological sample of a subject, wherein a measured value of ability to uptake sterol per unit volume of the biological sample is an indicator of diabetes onset risk.

In particular embodiments, the subject is a subject without a history of cardiovascular disease.

In particular embodiments, the biological sample is a blood sample.

In particular embodiments, the lipoprotein is high-density lipoprotein.

In particular embodiments, the measurement step is performed in a cell-free system.

In particular embodiments, the measurement step comprises the steps of: preparing lipoprotein incorporating labeled sterol by contacting the lipoprotein in the biological sample with the labeled sterol; and measuring ability to uptake the labeled sterol, based on the label of the labeled sterol incorporated into the lipoprotein.

In particular embodiments, the labeled sterol is the labeled cholesterol.

In particular embodiments, the labeled cholesterol is tagged cholesterol represented by the following formula (I):

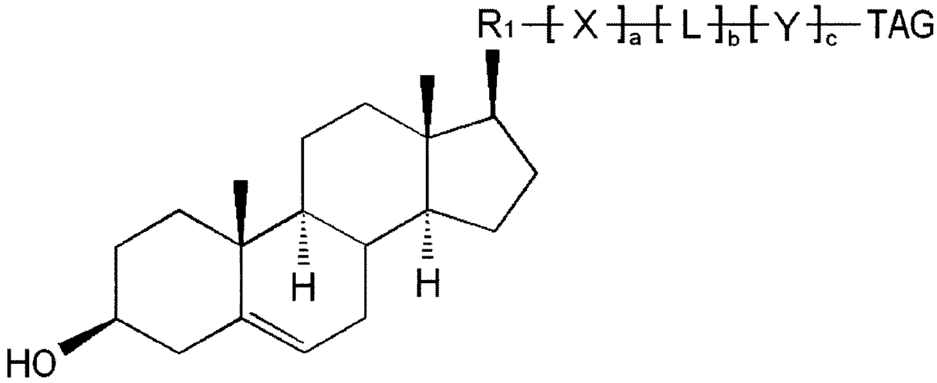

(I)

wherein $R_1$ is an alkylene group having 1 to 6 carbon atoms which may have a methyl group,
X and Y are identical or different, and are represented by $-R_2-NH-$, $-NH-R_2-$, $-R_2-(C=O)-NH-$, $-(C=O)-NH-R_2-$, $-R_2-NH-(C=O)-$, $-NH-(C=O)-R_2-$, $-R_2-(C=O)-$, $-(C=O)-R_2-$, $-R_2-(C=O)-O-$, $-(C=O)-O-R_2-$, $-R_2-O-(C=O)-$, $-O-(C=O)-R_2-$, $-R_2-(C=S)-NH-$, $-(C=S)-NH-R_2-$, $-R_2-NH-(C=S)-$, $-NH-(C=S)-R_2-$, $-R_2-O-$, $-O-R_2-$, $-R_2-S-$, or $-S-R_2-$, and each $R_2$ is independently an atomic bonding, an alkylene group having 1 to 10 carbon atoms which may have a substituent, or an arylene group or heteroarylene group having 6 to 12 carbon atoms which may have a substituent, or a cycloalkylene group or heterocycloalkylene group having 3 to 8 carbon atoms which may have a substituent;
L is represented by $-(CH_2)_d-[R_3-(CH_2)_e]_f-$ or $-[(CH_2)_e-R_3]_f-(CH_2)_d-$, and $R_3$ is an oxygen atom, a sulfur atom, -NH-, -NH-(C=O)- or -(C=O)-NH-;
TAG is a tag;
a and c are identical or different and are an integer of 0 to 6;
b is 0 or 1;
d and e are identical or different and are an integer of 0 to 12; and
f is an integer of 0 to 24.

In particular embodiments, the measurement step comprises the step of contacting the lipoprotein incorporating labeled sterol with a capture body that binds to the lipoprotein to form a complex containing the lipoprotein incorporating labeled sterol and the first capture body.

In particular embodiments, the capture body is immobilized on a solid phase, and the lipoprotein is immobilized on the solid phase.

In particular embodiments, the measurement step is performed by detecting a signal derived from the labeled sterol incorporated into the lipoprotein.

In particular embodiments, it is determined that the diabetes onset risk is high when the measured value of ability to uptake sterol per unit volume of the biological sample is less than or equal to a predetermined threshold, and that the diabetes onset risk is low when the measured value of ability to uptake sterol per unit volume of the biological sample is higher than the predetermined threshold.

The present invention provides the use of a reagent kit in a method for obtaining information on diabetes, comprising labeled sterol and a capture body that binds to lipoprotein.

The present invention provides an apparatus for determining diabetes onset risk, comprising a computer comprising a processor and a memory under control of the processor, wherein the memory is recorded with a computer program for causing the computer to execute the steps of: obtaining a measured value of lipoprotein's ability to uptake sterol in a biological sample of a subject; determining diabetes onset risk, based on the measured value of ability to uptake sterol per unit volume of the biological sample; and outputting a result of the determination.

The present invention provides a computer program for determining diabetes onset risk, which is recorded on a computer readable medium, and causes the computer to execute the following steps: obtaining a measured value of lipoprotein's ability to uptake sterol in a biological sample of a subject; determining diabetes onset risk, based on the measured value of ability to uptake sterol per unit volume of the biological sample; and outputting a result of the determination.

The present invention makes it possible to obtain a measured value of ability to uptake sterol per unit volume of a biological sample of a subject as a new indicator of diabetes onset risk.

BRIEF DESCRIPTION OF THE DRAWINGS

Fig. 1 is a view showing an example of an appearance of a reagent kit for obtaining information on diabetes.
Fig. 2 is a view showing an example of an appearance of a reagent kit for obtaining information on diabetes.
Fig. 3 is a schematic view showing an example of an apparatus for determining diabetes onset risk.
Fig. 4 is a block diagram showing a hardware configuration of the apparatus for determining diabetes onset risk.
Fig. 5 is a flowchart of determination using the apparatus for determining diabetes onset risk.
Fig. 6 is a flowchart of determination using the apparatus for determining diabetes onset risk.

DETAILED DESCRIPTION OF THE PREFERRED EMBODIMENTS

[1. Method for Obtaining Information on Diabetes]

In a method for obtaining information on diabetes according to this embodiment (hereinafter, also simply referred to as "method"), lipoprotein's ability to uptake cholesterol in a biological sample of a subject is measured.

(Subject)

The subject is not particularly limited as long as it is not diagnosed with diabetes. Examples of the subject include healthy persons, persons without a history of cardiovascular disease, and persons having environmental factors of diabetes onset such as hyperphagia, obesity and lack of exercise, and the like. Examples of the person without a history of cardiovascular disease include those who have not experienced any of myocardial infarction, cerebral infarction and coronary angioplasty.

(Biological sample)

The biological sample is not particularly limited as long as it is a sample containing lipoprotein of a subject. Examples of the biological sample include blood samples. Examples of the blood samples include blood (whole blood), plasma, serum and the like, with serum being particularly preferable.

The biological sample may be separated or fractionated by a known method such as ultracentrifugation or polyethylene glycol (PEG) precipitation to obtain a fraction containing a predetermined lipoprotein. In this embodiment, a fraction containing a predetermined lipoprotein prepared from a biological sample (hereinafter, also referred to as "lipoprotein fraction") can be used to measure ability to uptake sterol described later. As used herein, the term "biological sample" includes not only a biological sample collected from a subject, but also a lipoprotein fraction prepared from the biological sample. As the lipoprotein fraction, an HDL fraction is particularly preferred.

Lipoproteins may be any of HDL, low density lipoprotein (LDL), medium density lipoprotein (IDL), very low density lipoprotein (VLDL), and chylomicron (CM). HDL is lipoprotein with a density of 1.063 g/mL or more. LDL is lipoprotein with a density of 1.019 g/mL or more and less than 1.063 g/mL. IDL is lipoprotein with a density of 1.006 g/mL or more and less than 1.019 g/mL. VLDL is lipoprotein with a density of 0.95 g/mL or more and less than 1.006 g/mL. CM is lipoprotein with a density of less than 0.95 g/mL. In this embodiment, it is preferable to measure HDL's ability to uptake sterol.

Lipoproteins are known to esterify and incorporate cholesterol. When the labeled cholesterol described later is used as the labeled sterol, a fatty acid required for esterification reaction of cholesterol with lipoprotein or a composition containing the same (for example, liposome) may be added to a biological sample.

In this embodiment, the biological sample may be diluted and used. For example, in order to adjust lipoprotein concentration, a solution obtained by diluting the biological sample with an aqueous medium can be used for the measurement described later. Examples of the aqueous medium include buffers such as water, saline, phosphate buffered saline (PBS), and Tris-HCl.

In this embodiment, the biological sample may be diluted in the presence of a surfactant having no cyclic structure described later. For example, the biological sample may be diluted with a solution in which the surfactant having no cyclic structure is dissolved in the aqueous medium. By mixing the diluted biological sample with the labeled sterol, the lipoprotein and the labeled sterol come into contact in the presence of the surfactant having no cyclic structure.

The concentration of apolipoprotein that is a component of lipoprotein is an indicator of the concentration of lipoprotein in the biological sample. In this embodiment, the lipoprotein concentration in the biological sample may be adjusted by diluting the biological sample based on the obtained apolipoprotein concentration. The concentration of apolipoprotein can be measured by known immunoassays (for example, immunoturbidimetry). As the concentration of apolipoprotein, the concentration of ApoAI is particularly preferred. The measurement of apolipoprotein is preferably performed separately from the measurement of ability to uptake sterol, by taking a part of the biological sample to use it as a measurement sample.

In this embodiment, the biological sample may be diluted in the presence of cyclic oligosaccharides. For example,

the biological sample may be diluted with a solution in which cyclic oligosaccharides are dissolved in the aqueous medium. Examples of the cyclic oligosaccharides include cyclodextrin, hydroxypropyl cyclodextrin, and the like. The cyclic oligosaccharide functions as a blocking agent by including cholesterol derived from the biological sample.

In this embodiment, the biological sample may be diluted in the presence of a component that binds to a lipoprotein different from the lipoprotein to be measured. For example, the biological sample may be diluted with a solution in which the component is dissolved in the aqueous medium. Examples of the component that binds to a lipoprotein different from the lipoprotein to be measured include calixarene and the like. Calixarene binds to LDL, VLDL and CM but not to HDL. When measuring uptake ability by HDL, addition of calixarene to a sample can mask LDL, VLDL and CM derived from the sample.

In this embodiment, the biological sample may be diluted in the presence of a blocking agent to prevent nonspecific adsorption of the labeled sterol. For example, the biological sample may be diluted with a solution in which the blocking agent is dissolved in the aqueous medium. The addition of the blocking agent suppresses, for example, nonspecific adsorption of the labeled sterol to the solid phase or capture body described later. The blocking agent may be any component that can suppress adsorption of the labeled sterol to substances other than labeled cholesterol. Examples of a preferable blocking agent include 2-methacryloyloxyethyl phosphoryl choline type polymer. This polymer may be a homopolymer of 2-methacryloyloxyethyl phosphoryl choline or a copolymer with another monomer. As another monomer, an acrylic ester or methacrylic ester having an alkyl group having 1 to 20 carbon atoms as a hydrophobic group is preferable. The 2-methacryloyloxyethyl phosphoryl choline type polymer is commercially available. Examples thereof include a series of LIPIDURE (registered trademark) of NOF Corporation, and among them, LIPIDURE-BL203 is particularly preferable.

(Measurement method of cell-free system)

In this embodiment, it is preferable to measure lipoprotein’s ability to uptake sterol in a biological sample in a cell-free system. The cell-free system means that cells are not added for the purpose of being used to measure the lipoprotein’s ability to uptake sterol. A method for measuring the lipoprotein’s ability to uptake sterol in a cell-free system is itself known in the art and is described, for example, in US 2017/0315112 A1. Specifically, lipoprotein incorporating labeled sterol is prepared by contacting lipoprotein in a biological sample with the labeled sterol, and an ability to uptake labeled sterol is measured, based on a label of the labeled sterol incorporated into the lipoprotein. In this measurement method, since the labeled sterol is directly incorporated into the lipoprotein in the sample, it is not necessary to use cholesterol-accumulated cells such as macrophage as in the conventional measurement method of cholesterol efflux function. In this embodiment, even when the biological sample contains cells derived from the subject, it is considered that the cells themselves have little influence on the lipoprotein’s ability to uptake labeled sterol, and the measurement method is considered to be cell-free system.

(Labeled sterol)

The labeled sterol is sterol having a labeling substance. Hereinafter, the labeling substance possessed by the labeled sterol is also referred to as a "first label". Sterol used for the labeled sterol is not particularly limited as long as they are incorporated into lipoprotein. Examples of the sterol include cholesterol and derivatives thereof. Examples of the cholesterol derivatives include precursors of bile acids, precursors of steroids, and the like. Specifically, 3β-hydroxy-Δ5-cholenoic acid, 24-amino-5-colen-3β-ol and the like are preferable.

In this embodiment, the labeled sterol is preferably cholesterol having a labeling substance (hereinafter, also referred to as "labeled cholesterol"). A cholesterol moiety in the labeled cholesterol may have a structure of naturally occurring cholesterol, or a structure of cholesterol obtained by removing one or more methylene groups and/or methyl groups from an alkyl chain bonded to C17 position of naturally occurring cholesterol (also called norcholesterol).

It is preferable to use labeled cholesterol which is esterified by lipoproteins, since lipoproteins incorporate cholesterol by esterification. In this embodiment, the labeled cholesterol is esterified by lecithin-cholesterol acyltransferase (LCAT) of biological origin contained in the sample when it is mixed with the biological sample. Methods for confirming esterification of labeled cholesterol by lipoproteins themselves are known in the art and can be routinely performed by those skilled in the art.

The first label is not particularly limited as long as it is a labeling substance that makes it possible to detect the labeled sterol incorporated into lipoprotein. The first label may be, for example, a tag that is itself to be detected, or may be a substance that generates a detectable signal (hereinafter, also referred to as a "signal generating substance").

The tag as the first label is not particularly limited as long as it does not inhibit uptake of sterol by lipoproteins, and a substance capable of specifically binding to the tag is present or obtained. Hereinafter, sterol added with a tag as the first label is also referred to as a "tagged sterol". Similarly, cholesterol added with a tag as the first label is also referred to as a "tagged cholesterol". Tagged cholesterol itself is known in the art and is described, for example, in US

2017/0315112 A1.

Examples of the tagged cholesterol include tagged cholesterol represented by the following formula (I).

[Chemical Formula 1]

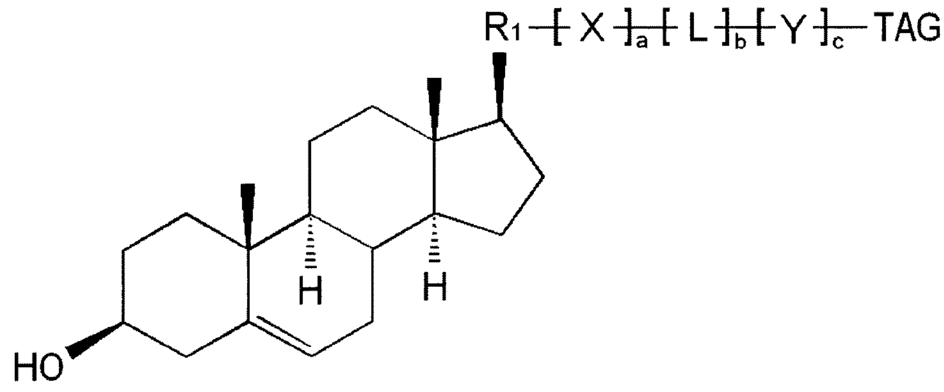

(I)

wherein $R_1$ is an alkylene group having 1 to 6 carbon atoms which may have a methyl group,

X and Y are identical or different, and are represented by $-R_2$-NH-, -NH-$R_2$-, - $R_2$-(C=O)-NH-, -(C=O)-NH-$R_2$-, -$R_2$-NH-(C=O)-, -NH-(C=O)-$R_2$-, -$R_2$-(C=O)-, - (C=O)-$R_2$-, -$R_2$-(C=O)-O-, -(C=O)-O-$R_2$-, -$R_2$-O-(C=O)-, -O-(C=O)-$R_2$-, -$R_2$-(C=S)-NH-, -(C=S)-NH-$R_2$-, -$R_2$-NH-(C=S)-, -NH-(C=S)-$R_2$-, -$R_2$-O-, -O-$R_2$-, -$R_2$-S-, or -S-$R_2$-, and each $R_2$ is independently an atomic bonding, an alkylene group having 1 to 10 carbon atoms which may have a substituent, or an arylene group or heteroarylene group having 6 to 12 carbon atoms which may have a substituent, or a cycloalkylene group or heterocycloalkylene group having 3 to 8 carbon atoms which may have a substituent;

L is represented by $-(CH_2)_d-[R_3-(CH_2)_e]_f-$ or $-[(CH_2)_e-R_3]_f-(CH_2)_d-$, and $R_3$ is an oxygen atom, a sulfur atom, -NH-, -NH-(C=O)- or -(C=O)-NH-;

TAG is a tag;

a and c are identical or different and are an integer of 0 to 6;

b is 0 or 1;

d and e are identical or different and are an integer of 0 to 12; and

f is an integer of 0 to 24.

In the formula (I), when a, b and c are all 0, the tagged cholesterol represented by the formula has no linker, and the tag and the cholesterol moiety are bonded directly. In the formula (I), when any one of a, b and c is not 0, the tagged cholesterol represented by the formula has a linker ($-[X]_a-[L]_b-[Y]_c-$) between the tag and the cholesterol moiety. It is believed that the linker further facilitates the bonding between the tag exposed on the outer surface of the lipoprotein and the capture body. Each substituent in the formula (I) will be described below.

$R_1$ may have an alkylene group having 1 to 6 carbon atoms as a main chain, and may have a methyl group at any position. $R_1$ corresponds to an alkyl chain bonded to C17 position of naturally occurring cholesterol. In this embodiment, when $R_1$ has 1 to 5 carbon atoms, $R_1$ preferably has a methyl group at the position of C20 in the naturally occurring cholesterol. When $R_1$ has 6 carbon atoms, $R_1$ preferably has the same structures as alkyl chains at C20 to C27 positions of the naturally occurring cholesterol.

$[X]_a$ corresponds to a connecting moiety between $R_1$ and L, $[Y]_c$ or the tag. $[Y]_c$ corresponds to a connecting moiety between $R_1$, $[X]_a$ or L and the tag. X and Y are determined according to the type of reaction that bonds between the cholesterol moiety and the linker and the type of reaction that bonds between the linker and the tag.

In $R_2$, the atomic bonding refers to a direct bonding without intervening any other atom. When $R_2$ is an alkylene group having 1 to 10 carbon atoms, examples of the alkylene group include methylene, ethylene, propylene, isopropylene, butylene, isobutylene, pentylene, neopentylene, hexylene, heptylene, octylene, 2-ethylhexylene, nonylene, and decylene groups. Among them, an alkylene group having 1 to 4 carbon atoms is preferred. When $R_2$ is an alkylene group having a substituent, the above number of carbon atoms does not include the number of carbon atoms in the substituent.

When $R_2$ is an arylene group or a heteroarylene group, the group should be an aromatic ring having 6 to 12 carbon atoms which may contain one or more hetero atoms selected from N, S, O, and P. Examples of the group include phenylene, naphthylene, biphenylylene, furanylene, pyrrolene, thiophenylene, triazolene, oxadiazolene, pyridylene, and pyrimidylene groups. When $R_2$ is an arylene group or a heteroarylene group having a substituent, the above number of carbon atoms does not include the number of carbon atoms in the substituent.

When $R_2$ is a cycloalkylene group or a heterocycloalkylene group, the group should be a non-aromatic ring

having 3 to 8 carbon atoms which may include one or more hetero atoms selected from N, S, O, and P. Examples of the group include cyclopropylene, cyclobutylene, cyclopentylene, cyclohexylene, cycloheptylene, cyclooctylene, pyrrolidinylene, piperidinylene, and morpholinylene groups. When $R_2$ is a cycloalkylene group or a heterocycloalkylene group having a substituent, the above number of carbon atoms does not include the number of carbon atoms in the substituent.

Examples of the substituent in $R_2$ include hydroxyl, cyano, alkoxy, =O, =S, - $NO_2$, -SH, halogen, haloalkyl, heteroalkyl, carboxyalkyl, amine, amide, and thioether groups. $R_2$ may have a plurality of the substituents. Halogen represents fluorine, chlorine, bromine, or iodine. Alkoxy represents an -O-alkyl group, and the alkyl group is a linear or branched saturated aliphatic hydrocarbon group having 1 to 5 carbon atoms, and preferably 1 or 2 carbon atoms.

Preferably, a and c are both 1, and X and Y are identical or different and are - (C=O)-NH- or -NH-(C=O)-.

L corresponds to a spacer and has a polymer structure that adds a predetermined length to the linker.

It is preferable that the polymer structural moiety does not inhibit the uptake of cholesterol by lipoproteins, and that the linker moiety is hardly incorporated into lipoproteins. The polymers include hydrophilic polymers such as polyethylene glycol (PEG). In a preferred embodiment, L is a structure represented by $-(CH_2)_d-[O-(CH_2)_e]_f-$ or $-[(CH_2)_e-O]_f-(CH_2)_d-$. d and e are identical or different and are an integer of 0 to 12, preferably an integer of 2 to 6, and more preferably both 2. f is an integer of 0 to 24, preferably an integer of 2 to 11, and more preferably an integer of 4 to 11.

The tag may be any of naturally occurring substances and synthesized substances, and examples thereof include compounds, peptides, proteins, nucleic acids, combinations thereof, and the like. The compound may be a labeling compound known in the art as long as a substance capable of specifically binding to the compound is present or obtained, and examples thereof include dye compounds and the like.

It is known in the art that cholesterol is esterified to increase its lipid solubility and promote its uptake by lipoproteins. The tag attached to cholesterol may be a lipid soluble or hydrophobic substance.

Examples of combinations of a tag and a substance capable of specifically binding to the tag include an antigen and an antibody that recognizes the antigen, a hapten and an anti-hapten antibody, a peptide or a protein and an aptamer that recognizes them, a ligand and a receptor thereof, oligonucleotides and oligonucleotides having complementary strands thereof, biotin and avidin (or streptavidin), histidine tag (a peptide containing histidine of 6 to 10 residues) and nickel, glutathione-S-transferase (GST) and glutathione, and the like. The antigen as a tag may be a peptide tag and a protein tag known in the art, and examples thereof include FLAG (registered trademark), hemagglutinin (HA), Myc protein, green fluorescent protein (GFP), and the like. Examples of the hapten as a tag include 2,4-dinitrophenol and the like.

Examples of the tagged sterol include sterols added with a structure represented by the following formula (II):

[Chemical Formula 2]

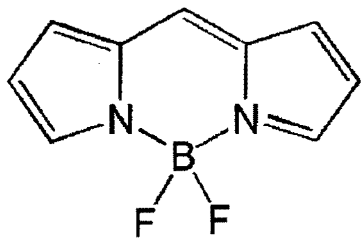

(II)

or a structure represented by the following formula (III):

[Chemical Formula 3]

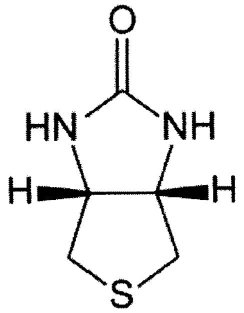

(III)

as a tag. The structure represented by the formula (II) is a boron dipyrromethene (BODIPY (registered trademark)) backbone, and the structure represented by the formula (III) shows a part of biotin. Sterol added with a structure represented by the following formula (II) or (III) as a tag is preferable since capture bodies for these tags are generally available. Sterol added with a 2,4-dinitrophenyl (DNP) group as a tag is also preferable because anti-DNP antibody is commercially available.

Examples of the tagged cholesterol having no linker include fluorescently labeled cholesterol (23-(dipyrrometheneboron difluoride)-24-norcholesterol, CAS No: 878557-19-8) represented by the following formula (IV).

[Chemical Formula 4]

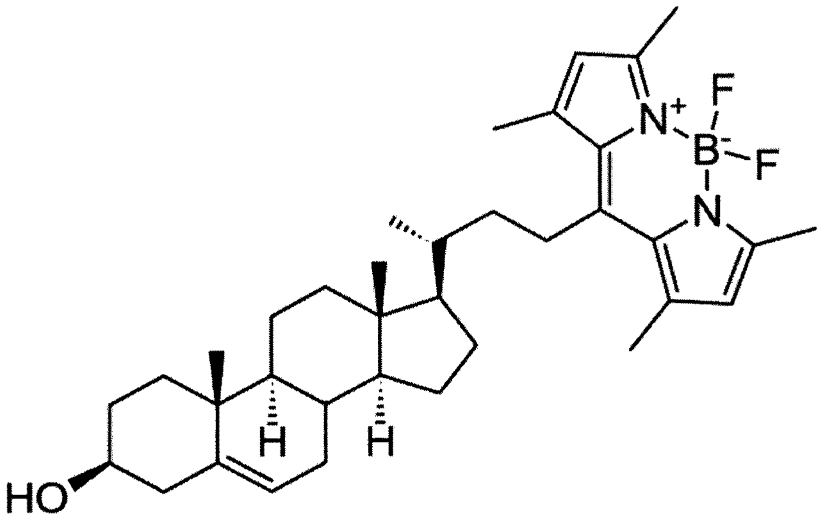

(IV)

This fluorescently labeled cholesterol is sold by Avanti Polar Lipids, Inc. under the trade name TopFluor Cholesterol. In the fluorescently labeled cholesterol represented by the formula (IV), the tag (the fluorescent moiety having BODIPY backbone structure) is directly bonded at C23 position of cholesterol. As a capture body that specifically binds to the fluorescent moiety having BODIPY backbone structure, an anti-BODIPY antibody (BODIPY FL Rabbit IgG Fraction, A-5770, Life technologies Corporation) is commercially available.

The tagged cholesterol in which the tag is bound via a linker includes a biotin-tagged cholesterol represented by the following formula (V).

[Chemical Formula 5]

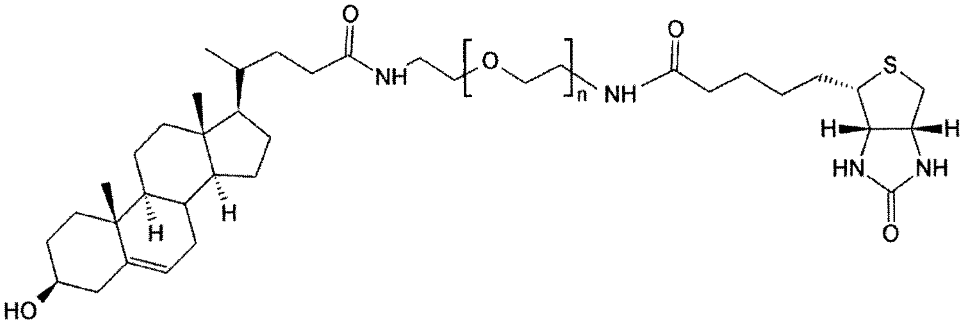

(V)

wherein, n is an integer of 0 to 24, preferably an integer of 2 to 11, and more preferably an integer of 4 to 11.

In the tagged cholesterol, the tag (the biotin moiety represented by the formula (III)) is bonded to the cholesterol moiety via the linker (polyethylene glycol). As a capture body that specifically binds to the biotin moiety, avidin or streptavidin is suitable. Avidin or streptavidin to which a labeling substance such as horseradish peroxidase (HRP) or alkaline phosphatase (ALP) is bound is also commercially available.

The tagged cholesterol in which the tag is bound via a linker includes DNP-added cholesterol represented by the following formula (VI).

[Chemical Formula 6]

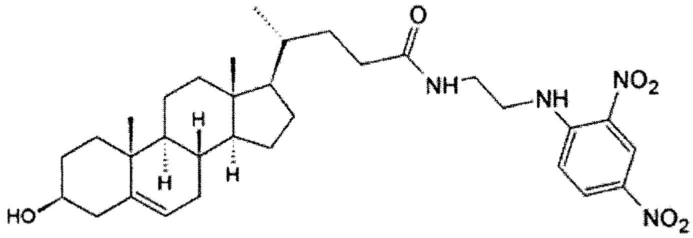

(VI)

In the tagged cholesterol, DNP is bound to the cholesterol moiety via a linker ($-(C{=}O)-NH-CH_2-CH_2-NH-$). As a capture body that specifically binds to DNP, an anti-DNP antibody is suitable. Anti-DNP antibodies to which a labeling substance such as HRP or ALP is bound is also commercially available.

Although the bonding form of the sterol moiety and the tag is not particularly limited, the sterol moiety and the tag may be bonded, or the sterol moiety and the tag may be bonded via a linker. Although the bonding means is not particularly limited, for example, a crosslink by utilizing a functional group is preferred because it is convenient. Although the functional group is not particularly limited, an amino group, a carboxyl group and a sulfhydryl group are preferred because commercially available crosslinkers can be used.

Since cholesterol has no functional group in the alkyl chain bonded to the C17 position, a cholesterol derivative having a functional group in the alkyl chain is preferably used in the addition of the tag. Examples of the cholesterol derivative include precursors of bile acid, precursor of steroids, and the like. Specifically, 3β-hydroxy-Δ5-cholenoic acid, 24-amino-5-colen-3β-ol and the like are preferable. The functional group of the tag varies depending on the type of the tag. For example, when a peptide or a protein is used as the tag, an amino group, a carboxyl group and a sulfhydryl group (SH group) can be used. When biotin is used as the tag, a carboxyl group in the side chain can be used. The linker is preferably a polymer compound having functional groups at both terminals. When biotin is added as the tag, a commercially available biotin labeling reagent may be used. The reagent contains biotin to which various length of spacer arms (for example, PEG) having a functional group such as an amino group are bound at the terminal.

Examples of the signal generating substance as the first label include fluorescent substances, luminescent substances, color-developing substances, radioactive isotopes, and the like. Among them, fluorescent substances are particularly preferred. Preferably, the fluorescent substance contains a fluorophore having a polar structure. In the art, the fluorescent substance containing a fluorophore having a polar structure itself is known.

Sterol labeled with a signal generating substance can be produced by adding the substance to sterol by a known method. In sterol, a position to which a signal generating substance is added is not particularly limited, and can be appropriately determined according to the type of signal generating substance to be used. Although the bonding form of the sterol and the signal generating substance is not particularly limited, it is preferable that both are directly bonded via a covalent bond.

Examples of the sterol containing a fluorescent substance include a fluorescently labeled sterol containing a

fluorophore having a BODIPY backbone structure represented by the formula (II). In this embodiment, commercially available fluorescently labeled sterol may be used. Examples of the sterol containing a fluorophore having a BODIPY backbone structure include fluorescently labeled cholesterol represented by the formula (IV). The fluorescently labeled cholesterol represented by the formula (IV) generates a fluorescence signal at a wavelength of 525 to 550 nm by an excitation wavelength of 470 to 490 nm.

(Preparation of lipoprotein incorporating labeled sterol)

In this embodiment, contact between lipoprotein in a biological sample and labeled sterol can be performed, for example, by mixing the biological sample with a solution containing labeled sterol. After mixing them, the lipoprotein starts to incorporate the labeled sterol, and lipoprotein incorporating labeled sterol is obtained. The conditions of temperature and time in mixing are not particularly limited. For example, a mixed solution of a biological sample and labeled sterol may be incubated at 20 to 48°C, preferably 25 to 42°C, for 1 minute to 24 hours, preferably 10 minutes to 2 hours. During the incubation, the mixed solution may be allowed to stand, or may be stirred or shaken.

The addition amount of the labeled sterol is not particularly limited, but may be added in a slight excess so that the labeled sterol is not depleted. For example, the labeled sterol can be added to a final concentration of 0.1 μM or more and 30 μM or less, and preferably 1 μM or more and 10 μM or less.

(Surfactant having no cyclic structure)

In this embodiment, lipoprotein incorporating labeled sterol may be prepared, in the presence of a surfactant having no cyclic structure. The surfactant having no cyclic structure refers to a surfactant which is an acyclic compound. In this case, the contact between lipoprotein in a sample and labeled sterol can be performed, for example, by mixing the biological sample, a solution containing labeled sterol, and a solution containing a surfactant having no cyclic structure. The order of mixing is not particularly limited. Alternatively, a solution containing a surfactant having no cyclic structure and labeled sterol (hereinafter, also referred to as a "reaction buffer") may be prepared in advance, and this reaction buffer may be mixed with the biological sample.

By performing the mixing in the presence of the surfactant having no cyclic structure, an uptake reaction by lipoprotein is promoted. The surfactant having no cyclic structure also plays the role of blocking agent. Therefore, an animal-derived protein generally used as a blocking agent in immunological measurements, such as bovine serum albumin (BSA), may not be used. The animal-derived protein may not be stable in lot-to-lot quality. The animal-derived protein tends to non-specifically bind to components in the biological sample, antibodies added to a measurement system, labeled sterols and the like, as compared to the surfactant. Since the surfactant having no cyclic structure is a synthetic product, quality is almost constant and influence on the measurement is minor compared to the animal-derived protein.

The surfactant having no cyclic structure can be appropriately selected from nonionic surfactants, cationic surfactants, anionic surfactants and amphoteric surfactants, but is preferably a nonionic surfactant.

Nonionic surfactants having no cyclic structure are not particularly limited, and examples thereof include polyalkylene glycol ethylene oxide adduct, polyoxyethylene alkyl ether, polyoxyethylene polyoxypropylene alkyl ether, polyoxyalkylene dialkyl ether, polyoxyalkylene alkenyl ether, polyoxyethylene branched alkyl ether, polyoxyethylene polyoxypropylene alkyl ether, polyoxyalkylene ether, polyethylene glycol fatty acid ester, glycerin fatty acid ester, polyglycerol fatty acid ester, propylene glycol fatty acid ester, polypropylene glycol fatty acid ester, polyoxyethylene coconut fatty acid glyceryl, polyoxyethylene hardened castor oil, polyoxyethylene castor oil, polyoxyethylene triisostearic acid, polyoxyethylene sorbitol fatty acid ester, polyoxyethylene alkylamine, polyoxyethylene polyoxypropylene alkylamine, polyoxyethylene alkyl propylene diamine, fatty acid diethanolamide, polyoxyethylene fatty acid monoethanolamide, and the like. The nonionic surfactant having no cyclic structure may be used alone or in combination of two or more.

Among the nonionic surfactants, polyoxyethylene alkyl ether and polyalkylene glycol ethylene oxide adduct are preferable. Examples of the polyoxyethylene alkyl ether include polyoxyethylene lauryl ether, polyoxyethylene cetyl ether, polyoxyethylene stearyl ether, polyoxyethylene behenyl ether, and the like. In this embodiment, polyoxyethylene lauryl ether is particularly preferred. Polyoxyethylene lauryl ether is commercially available, and examples thereof include NONION K-230 and NONION K-220 of NOF Corporation, EMULGEN 123P and EMULGEN 130K of Kao Corporation, and the like.

In this embodiment, as the polyalkylene glycol ethylene oxide adduct, a block copolymer is preferable, and a triblock copolymer of ethylene oxide and propylene oxide is more preferable. As a nonionic surfactant of the block copolymer, a compound represented by the following formula (VII) is particularly preferable.

[Chemical Formula 7]

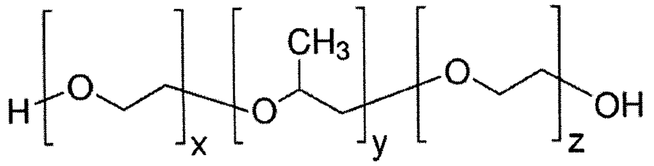

(VII)

wherein x, y and z are identical or different and an integer of 1 or more, and
the molecular weight of the polypropylene oxide moiety is 2750 or less.

The compounds of the formula (VII) are also called polypropylene glycol ethylene oxide adducts, polyoxyethylene polyoxypropylene glycols, polyoxyethylene polyoxypropylene block polymers, or pluronic (trademark)-type nonionic surfactants.

In the formula (VII), the molecular weight of the polypropylene oxide moiety (hereinafter also referred to as "PPO") refers to a molecular weight of a moiety represented by $-(O-CH(CH_3)-CH_2)_y-$, and a molecular weight calculated from the molecular structural formula. In this embodiment, the molecular weight of PPO is preferably 900 or more and 2750 or less, more preferably 950 or more and 2750 or less, and particularly preferably 950 or more and 2250 or less. Because PPO in pluronic-type nonionic surfactants is relatively highly hydrophobic, the larger the molecular weight of PPO, the greater the hydrophobicity of the surfactant. Therefore, a pluronic-type nonionic surfactant having a molecular weight of PPO more than 2750 may act on lipoprotein to inhibit uptake of cholesterol.

The average molecular weight of the compound represented by the formula (VII) is preferably 1000 to 13000. In the formula (VII), the sum of x and z is preferably 2 to 240. In the formula (VII), y is preferably 15 to 47, more preferably 16 to 47, and particularly preferably 16 to 39. As used herein, the "average molecular weight" is a weight average molecular weight measured by gel permeation chromatography (GPC).

Pluronic-type nonionic surfactants are commercially available, and examples thereof include the Pluronic series of BASF, the Pronon series of NOF Corporation, the Adeka Pluronic series of Asahi Denka Co., Ltd., and the like. The Pluronic series is classified by a Pluronic grid in which the molecular weight of PPO is taken on the vertical axis and the ethylene oxide (EO) content (%) is taken on the horizontal axis (for example, see Pitto-Barry A. and Barry N.P.E., Poly. Chem., 2014, vol. 5, p. 3291-3297). The Pluronic grid is provided not only from academic literature but also from various manufacturers. In this embodiment, the compound represented by the formula (VII) may be selected based on the pluronic grid.

Examples of the pluronic-type nonionic surfactants represented by the formula (VII) include Pluronic L31 (molecular weight of PPO: 950, EO content: 10%), Pluronic L35 (molecular weight of PPO: 950, EO content: 50%), Pluronic F38 (molecular weight of PPO: 950, EO content: 80%), Pluronic L42 (molecular weight of PPO: 1200, EO content: 20%), Pluronic L43 (molecular weight of PPO: 1200, EO content: 30%), Pluronic L44 (molecular weight of PPO: 1200, EO content: 40%), Pluronic L61 (molecular weight of PPO: 1750, EO content: 10%), Pluronic L62 (molecular weight of PPO: 1750, EO content: 20%), Pluronic L63 (molecular weight of PPO: 1750, EO content: 30%), Pluronic L64 (molecular weight of PPO: 1750, EO content: 40%), Pluronic P65 (molecular weight of PPO: 1750, EO content: 50%), Pluronic F68 (molecular weight of PPO: 1750, EO content: 80%), Pluronic L72 (molecular weight of PPO: 2050, EO content: 20%), Pluronic P75 (molecular weight of PPO: 2050, EO content: 50%), Pluronic F77 (molecular weight of PPO: 2050, EO content: 70%), Pluronic L81 (molecular weight of PPO: 2250, EO content: 10%), Pluronic P84 (molecular weight of PPO: 2250, EO content: 40%), Pluronic P85 (molecular weight of PPO: 2250, EO content: 50%), Pluronic F87 (molecular weight of PPO: 2250, EO content: 70%), Pluronic F88 (molecular weight of PPO: 2250, EO content: 80%), Pluronic L92 (molecular weight of PPO: 2750, EO content: 20%), Pluronic P94 (molecular weight of PPO: 2750, EO content: 40%), Pluronic F98 (molecular weight of PPO: 2750, EO content: 80%), and the like.

Alternatively, the triblock copolymer of ethylene oxide and propylene oxide may be a compound represented by the following formula (VIII). This compound is also referred to as a reverse pluronic-type nonionic surfactant.

[Chemical Formula 8]

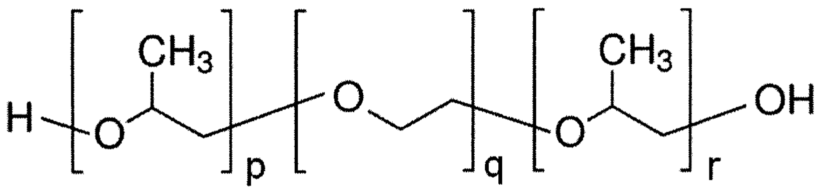 (VIII)

wherein p, q and r are identical or different and an integer of 1 or more, and the molecular weight of the polypropylene oxide moiety is 2750 or less.

In the formula (VIII), the molecular weight of the polypropylene oxide moiety refers to a molecular weight of a moiety represented by $-(O-CH(CH_3)-CH_2)_p-$ and $-(O-CH(CH_3)-CH_2)_r-$, and a molecular weight calculated from the molecular structural formula. In this embodiment, the molecular weight of PPO is preferably 900 or more and 2750 or less, and more preferably 950 or more and 2250 or less. Pluronic-type nonionic surfactants represented by the formula (VIII) are commercially available, and examples thereof include Pluronic 10R5 (molecular weight of PPO: 950, EO content: 50%) of BASF and the like.

The average molecular weight of the compound represented by the formula (VIII) is preferably 1000 to 13000. In the formula (VIII), q is preferably 2 to 240. In the formula (VIII), the sum of p and r is preferably 15 to 47, more preferably 16 to 47, and particularly preferably 16 to 39. As used herein, the "average molecular weight" is a weight average molecular weight measured by GPC.

(Formation of complex of lipoprotein and first capture body)

In the method of this embodiment, it is preferable to include a step of contacting lipoprotein incorporating labeled sterol with a first capture body that binds to the lipoprotein to form a complex containing the lipoprotein incorporating labeled sterol and the first capture body. The contact between the lipoprotein and the first capture body causes the first capture body to bind to the lipoprotein to form a complex of the lipoprotein and the first capture body.

The first capture body is not particularly limited as long as it is a substance capable of specifically binding to a part of the surface of lipoprotein. As the first capture body, an antibody that specifically binds to lipoprotein is preferable, and an antibody that can specifically bind to apolipoprotein that is a component of lipoprotein is more preferable. Examples of the antibody include anti-ApoAI antibodies, anti-ApoAII antibodies, and the like. Among them, anti-ApoAI antibodies are particularly preferred. Commercially available anti-lipoprotein antibodies and anti-ApoAI antibodies may be used.

The anti-lipoprotein antibodies and the anti-ApoAI antibodies may be monoclonal antibodies or polyclonal antibodies. The origin of the antibody is not particularly limited, and may be an antibody derived from any mammal such as a mouse, a rat, a hamster, a rabbit, a goat, a horse, or a camel. The isotype of antibody may be any of IgG, IgM, IgE, IgA and the like and is preferably IgG. In this embodiment, a fragment of an antibody and a derivative thereof may be used as the first capture body, and examples thereof include Fab fragments, F(ab')2 fragments, and the like.

The contact between the lipoprotein and the first capture body can be performed before the contact between the lipoprotein and the labeled sterol, substantially simultaneously with the contact between the lipoprotein and the labeled sterol, or after the contact between the lipoprotein and the labeled sterol. The contact between the lipoprotein and the first capture body can be performed, for example, by mixing a biological sample, a solution containing labeled sterol, and a solution containing the first capture body. The mixing may be performed in the presence of a surfactant having no cyclic structure. The order of mixing the biological sample, the labeled sterol, and the first capture body is not particularly limited. These may be mixed substantially simultaneously or may be sequentially mixed. The addition amount of the first capture body is not particularly limited, and can be appropriately set by those skilled in the art according to the type of the first capture body and the like.

In a preferred embodiment, the contact between the lipoprotein and the first capture body is performed after the contact between the lipoprotein and the labeled sterol. As a result, a complex of the lipoprotein incorporating labeled sterol and the first capture body is formed. For example, first, a sample containing lipoprotein and a solution containing labeled sterol are mixed to prepare lipoprotein incorporating labeled sterol. A solution containing the first capture body is added thereto and mixed. In this case, after the lipoprotein incorporated the labeled sterol, a complex of the lipoprotein and the first capture body is formed.

When an anti-ApoAI antibody is used as the first capture body, the lipoprotein may be treated with an oxidizing agent before the anti-ApoAI antibody and the lipoprotein incorporating labeled sterol come in contact with each other.

By the action of the oxidizing agent, reactivity of the anti-ApoAI antibody and the lipoprotein can be improved. Examples of the oxidizing agent include hydrogen peroxide, peroxynitrite, chlorine dioxide, hypochlorous acid, and the like.

There are no particular limitations on the conditions of temperature and time in the contact between the lipoprotein and the first capture body. For example, the mixed solution may be incubated at 20 to 48°C, preferably 25 to 42°C, for 1 minute to 24 hours, preferably 10 minutes to 2 hours. During the incubation, the mixed solution may be allowed to stand, or may be stirred or shaken.

In this embodiment, a complex of the lipoprotein and the first capture body may be formed on a solid phase. For example, a biological sample, a labeled sterol solution, a solution containing the first capture body, and a solid phase may be mixed. After mixing the sample containing lipoprotein, the labeled sterol solution, and the solution containing the first capture body, the obtained mixed solution may be mixed with the solid phase. Alternatively, the first capture body may be preliminarily immobilized on the solid phase. For example, a complex is formed on a solid phase by adding a solid phase on which an anti-lipoprotein antibody is immobilized to a mixed solution of a sample containing lipoprotein and a labeled sterol solution. The mixing may be performed in the presence of a surfactant having no cyclic structure. The conditions of temperature and time when using a solid phase are not particularly limited. For example, the conditions may be similar to those for the contact between the lipoprotein and the first capture body.

The solid phase is preferably a solid phase capable of capturing the first capture body in the complex. The type of the solid phase is not particularly limited, and examples of the solid phase include a solid phase of a material that physically adsorbs the antibody, a solid phase on which a molecule that specifically binds to the antibody is immobilized, and the like. Examples of the molecule that specifically binds to the antibody include protein A or G, an antibody (i.e., a secondary antibody) that specifically recognizes an antibody, and the like. A combination of substances interposed between the antibody and the solid phase can be used to bind them. Examples of such a combination of substances include combinations of biotin and avidin (or streptavidin), haptens and anti-hapten antibodies, and the like. For example, when the first capture body is previously modified with biotin, the first capture body can be captured by a solid phase on which avidin or streptavidin is immobilized.

The solid phase material can be selected from organic polymer compounds, inorganic compounds, biopolymers, and the like. Examples of the organic polymer compounds include latex, polystyrene, polypropylene, styrene-methacrylic acid copolymer, styrene-glycidyl (meth)acrylate copolymer, styrene-styrene sulfonate copolymer, methacrylic acid polymer, acrylic acid polymer, acrylonitrile butadiene styrene copolymer, vinyl chloride-acrylate copolymer, polyvinyl acetate acrylate, and the like. Examples of the inorganic compounds include magnetic bodies (iron oxide, chromium oxide, cobalt, ferrite, etc.), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include particles, microplates, microtubes, test tubes, and the like. Among them, microplates and particles are preferred, and 96-well microplates and magnetic particles are particularly preferred.

(Washing step)

In this embodiment, a washing step of removing unreacted free components may be performed between the contact between the lipoprotein and the labeled sterol and the measurement of ability to uptake sterol described later. This washing step includes B/F separation and washing of the complex. The unreacted free components are components that do not constitute a complex containing lipoprotein incorporating labeled sterol. Examples thereof include free labeled sterol that has not been incorporated into lipoprotein, free first capture body that has not bound to lipoprotein, and the like. The means for B/F separation is not particularly limited, but the complex and the unreacted free components can be separated by recovering only the complex by, for example, ultracentrifugation. In a case where the complex is formed on a solid phase, when the solid phase is particles, the complex and the unreacted free components can be separated by recovering the particles by centrifugation or magnetic separation, and removing the supernatant. When the solid phase is a container such as a microplate or a microtube, the complex and the unreacted free components can be separated by removing a liquid containing the unreacted free components.

After removing the unreacted free components, the recovered complex can be washed with a suitable aqueous medium. Examples of the aqueous medium include water, saline, buffers such as PBS and Tris-HCl, and the like. In this embodiment, the washing step is preferably performed in the presence of a surfactant having no cyclic structure. For example, a surfactant having no cyclic structure may be dissolved in the aqueous medium to prepare a washing solution, and the recovered complex may be washed with the washing solution. When a solid phase is used, the solid phase that has captured the complex may be washed with a washing solution. Specifically, a washing solution is added to the recovered complex or the solid phase that has captured the complex, and B/F separation is performed again.

(Measurement of ability to uptake sterol)

In the method of this embodiment, after preparing lipoprotein incorporating labeled sterol, an ability to uptake

labeled sterol by lipoproteins is measured. Measurement of ability to uptake sterol is performed by detecting a signal derived from labeled sterol incorporated into lipoprotein. Since this signal reflects the amount of labeled sterol incorporated into lipoprotein, a detection result of the signal is an indicator of lipoprotein's ability to uptake sterol.

The phrase "detecting a signal" herein includes qualitatively detecting the presence or absence of a signal, quantifying a signal intensity, and semi-quantitatively detecting the intensity of a signal in a plurality of stages, such as "not generating signal", "weak", and "strong". In this embodiment, it is preferable to quantify the signal intensity to obtain a measured value.

The signal derived from the labeled sterol incorporated into lipoprotein may be a signal directly generated from the labeled sterol. The signal can be detected, for example, when using labeled sterol having a signal generating substance as the first label. That is, the uptake ability can be measured by detecting the signal generated from the first label in the labeled sterol incorporated into lipoprotein. For example, in the case of using a fluorescently labeled sterol, a fluorescence intensity should be measured. Methods for measuring fluorescence intensity themselves are known in the art. For example, a fluorescence intensity generated from the complex can be measured by using known measuring devices such as a spectrofluorimeter and a fluorescence plate reader. The excitation wavelength and the fluorescence wavelength can be appropriately determined according to the type of fluorescently labeled cholesterol used. For example, when the fluorescently labeled cholesterol of the formula (IV) is used, the excitation wavelength should be determined from the range of 470 to 490 nm, and the fluorescence wavelength should be determined from the range of 525 to 550 nm.

(Formation of complex of labeled sterol and second capture body)

The signal derived from the labeled sterol incorporated into lipoprotein may be a signal generated when detecting the labeled sterol incorporated into lipoprotein. In order to detect the labeled sterol incorporated into lipoprotein, in this embodiment, the labeled sterol may be contacted with a second capture body that binds to the labeled sterol to form a complex. In this case, the labeled sterol is preferably tagged sterol, and more preferably tagged cholesterol. The second capture body is preferably a substance that specifically binds to the tag.

The detection principle of the tagged cholesterol incorporated into lipoprotein is as follows. When cholesterol is incorporated into lipoprotein, the cholesterol normally migrates from a surface layer to a central part of the lipoprotein particle. In the tagged cholesterol, it is the cholesterol moiety that is incorporated into lipoprotein, and the tag is considered to be exposed on an outer surface of the lipoprotein. The "outer surface of the lipoprotein" refers to the outer surface of the lipoprotein particles. The "exposed on an outer surface" means that both existing on the outer surface of the lipoprotein and protruding from the outer surface of the lipoprotein. In this embodiment, the tag exposed on the outer surface is brought into contact with a second capture body that specifically binds to the tag to form a complex. Then, cholesterol incorporated into lipoprotein is detected by detecting the second capture body in this complex.

The substance that specifically binds to the tag can be appropriately determined according to the type of the tag. For example, referring to a combination of the above-mentioned tag and a substance capable of specifically binding to the tag, the substance can be selected from an antibody, a ligand receptor, an oligonucleotide, biotin, avidin (or streptavidin), a histidine tag or nickel, GST, glutathione, and the like. Among them, an antibody that specifically binds to the tag is preferred. The antibody may be a commercially available antibody or an antibody prepared by a method known in the art. The antibody may be a monoclonal antibody or a polyclonal antibody. The origin and isotype of the antibody are not particularly limited, and are similar to those described for the anti-HDL antibody. Fragments of antibodies and derivatives thereof may be used, and examples thereof include Fab fragments, F(ab')2 fragments, and the like.

In this embodiment, it is preferable to contact the labeled sterol and the second capture body, after forming a complex of the lipoprotein incorporating labeled sterol and the first capture body. In particular, it is preferable to contact the labeled sterol and the second capture body, after forming a complex of the lipoprotein incorporating labeled sterol and the first capture body on the solid phase. As a result, a complex of the first capture body, the lipoprotein incorporating labeled sterol and the second capture body is formed. In this complex, the lipoprotein incorporating labeled sterol is sandwiched between the first capture body and the second capture body. In this embodiment, the complex of the first capture body, the lipoprotein incorporating labeled sterol and the second capture body is also referred to as a "sandwich complex".

The conditions of temperature and time in the contact between the labeled sterol and the second capture body are not particularly limited. For example, a mixture of a solution containing the complex of the lipoprotein and the first capture body and a solution containing the second capture body may be incubated at 4 to 60°C, preferably 25 to 42°C, for 1 second to 24 hours, preferably 10 minutes to 2 hours. During the incubation, the mixture may be allowed to stand, or may be stirred or shaken.

(Second label)

The second capture body is preferably labeled with a second label. When the second capture body is labeled

with a second label, ability to uptake sterol can be measured by detecting a signal derived from the second label in the complex of the labeled sterol and the second capture body.

The second label may be a signal generating substance, or a substance that catalyzes a reaction of other substances to generate a detectable signal can be used. Examples of the signal generating substance include fluorescent substances, radioactive isotopes, and the like. Examples of the substance that catalyzes the reaction of other substances to generate a detectable signal include enzymes. Examples of the enzyme include alkaline phosphatase, peroxidase, β-galactosidase, glucose oxidase, tyrosinase, acid phosphatase, luciferase, and the like. Examples of the fluorescent substances include fluorescent dyes such as fluorescein isothiocyanate (FITC), rhodamine and Alexa Fluor (registered trademark) and cyanine dyes, fluorescent proteins such as GFP, and the like. Examples of the radioactive isotopes include $^{125}I$, $^{14}C$, $^{32}P$, and the like. Among them, an enzyme is preferable, and alkaline phosphatase and peroxidase are particularly preferable.

In this embodiment, labeling of the second capture body with the second label can be performed by directly or indirectly binding the second label to the second capture body. For example, the second label can be directly bonded to the second capture body using a commercially available labeling kit or the like. The second label may be indirectly bound to the second capture body by using a secondary antibody obtained by labeling an antibody capable of specifically binding to the second capture body with the second label. In this embodiment, a second capture body to which a second label is bound may be used, or a second capture body and a secondary antibody having a second label may be used.

In this embodiment, the washing step of removing unreacted free components may be performed before signal detection. Examples of the unreacted free components include a free second capture body that did not bind to the tag, a free second label that did not bind to the second capture body, and the like. Specific washing method and washing solution are similar to those in the above-mentioned washing step.

(Detection of signal derived from second label)

Methods for detecting a signal derived from the second label themselves are known in the art. In this embodiment, a suitable measurement method can be selected according to the type of signal derived from the second label. For example, when the second label is an enzyme, signals such as light and color generated by reacting an enzyme and a substrate for the enzyme can be measured by using a known apparatus. Examples of the measuring device include a spectrophotometer, a luminometer, and the like.

The substrate of the enzyme can be appropriately selected from known substrates according to the type of the enzyme. For example, when peroxidase is used as the enzyme, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB). When alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate.

When the second label is a radioactive isotope, radiation as a signal can be measured using a known apparatus such as a scintillation counter. When the second label is a fluorescent substance, fluorescence as a signal can be measured using a known apparatus such as a fluorescence microplate reader. The excitation wavelength and the fluorescence wavelength can be appropriately determined according to the type of fluorescent substance used.

(Measurement of amount of lipoprotein immobilized on solid phase)

In this embodiment, the amount of lipoprotein immobilized on a solid phase (hereinafter, also referred to as "the amount of lipoprotein captured") may be measured, as necessary. This measurement may be performed separately from the measurement of ability to uptake sterol, or may be performed using a solid phase after the measurement of ability to uptake sterol. When the measurement is performed separately from the measurement of ability to uptake sterol, a plurality of solid phases on which lipoprotein incorporated with labeled sterol are immobilized are prepared. Then, at least one solid phase is used to measure the amount of lipoprotein captured, and the remaining solid phase is used to measure ability to uptake sterol.

The amount of lipoprotein captured itself can be measured based on a principle of known ELISA method. For example, it is possible to measure the amount of lipoprotein captured by contacting lipoprotein immobilized on a solid phase with a third capture body that binds to the lipoprotein to form a complex, and detecting the complex. The third capture body is not particularly limited as long as it is a substance capable of specifically binding to a part of the surface of lipoprotein. The details of the third capture body are similar to those described for the first capture body. The third capture body may be the same as or different from the first capture body. A preferred third capture body is an anti-ApoAI

antibody. When the first capture body and the third capture body are antibodies that bind to the same antigen (for example, ApoAI), each other's epitopes are preferably different.

The third capture body is preferably labeled with a third label. When the third capture body is labeled with a third label, the amount of lipoprotein captured can be measured by detecting a signal derived from the third label in the complex of the lipoprotein immobilized on a solid phase and the third capture body. The details of the third label are similar to those described for the second label. The third label may be the same as or different from the second label. In this embodiment, the amount of lipoprotein captured can be measured by detecting the signal derived from the third label. The method of detecting the signal derived from the third label is similar to that described for the second label.

(Measured value of ability to uptake sterol per unit volume of biological sample)

In this embodiment, the measurement result of lipoprotein's ability to uptake sterol can be obtained as information on subject's diabetes. Specifically, the measured value of ability to uptake sterol per unit volume of the biological sample of the subject serves as an indicator of diabetes onset risk. The measured value of ability to uptake sterol per unit volume of the biological sample can be obtained as follows. For example, when the ability to uptake sterol is measured without diluting a predetermined amount of a biological sample (or a lipoprotein fraction prepared from a predetermined amount of a biological sample), the obtained measured value itself can be obtained as the measured value of ability to uptake sterol per unit volume of the biological sample. Alternatively, a value obtained by dividing the obtained measured value by the volume (value of a predetermined amount) of the biological sample used for measurement may be obtained as the measured value of ability to uptake sterol per unit volume of the biological sample.

When the biological sample is diluted based on the concentration of apolipoprotein in the biological sample and used for measurement, it is desirable that the obtained measured value of ability to uptake sterol is converted to the measured value of ability to uptake sterol per unit volume of the biological sample. This is because the concentration of lipoprotein in the biological sample is adjusted by dilution. For example, when the biological sample is diluted based on the concentration of apolipoprotein in the biological sample and used for measurement, the obtained measured value of ability to uptake sterol can be converted to the measured value of ability to uptake sterol per unit volume of the biological sample, by the following equation (1).

$$\text{(Measured value of ability to uptake sterol per unit volume of biological sample)} = [\text{(Measured value of ability to uptake sterol)}/\text{(Measured value of amount of lipoprotein captured)}] \times \text{(Measured value of concentration of apolipoprotein in biological sample)} \ldots \quad (1)$$

In the above equation (1), the measured value of ability to uptake sterol per lipoprotein is obtained by dividing the measured value of ability to uptake sterol by the measured value of the amount of lipoprotein captured. As mentioned above, the concentration of apolipoprotein in the biological sample is an indicator of the concentration of lipoprotein in the biological sample. Therefore, the measured value of ability to uptake sterol per unit volume of the biological sample can be obtained by multiplying the measured value of ability to uptake sterol per lipoprotein by the measured value of the concentration of apolipoprotein in the biological sample.

The measured value of ability to uptake sterol per unit volume of the biological sample can be used to determine diabetes onset risk of a subject. A medical worker such as a doctor may determine diabetes onset risk using the measured value, or may determine diabetes onset risk by combining the measured value with other information. The "other information" includes blood glucose level, HbAlc measured value, and other medical findings.

[2. Method for Determining Diabetes Onset Risk of Subject]

In this embodiment, the result of lipoprotein's ability to uptake sterol obtained by the above method can be used to determine diabetes onset risk of a subject. In the method for determining diabetes onset risk of a subject according to this embodiment (hereinafter also referred to as "determination method"), first, lipoprotein's ability to uptake sterol in a biological sample collected from the subject is measured. The details of this measurement are the same as those described for the method for obtaining information on diabetes of this embodiment.

In the determination method of this embodiment, the diabetes onset risk of the subject is determined based on a comparison result of the measured value of ability to uptake sterol per unit volume of the biological sample and the

predetermined threshold. Specifically, the following determination (1) and/or (2) is made:

(1) When the measured value of ability to uptake sterol per unit volume of the biological sample is less than or equal to a predetermined threshold, it is determined that the diabetes onset risk is high;
(2) When the measured value of ability to uptake sterol per unit volume of the biological sample is higher than the predetermined threshold, it is determined that the diabetes onset risk is low.

In the above determination, when the measured value of ability to uptake sterol per unit volume of the biological sample is equal to the predetermined threshold, it has been determined that the diabetes onset risk is high, but it may be determined that the diabetes onset risk is low. That is, the following determination (1) and/or (2) may be made:

(1) When the measured value of ability to uptake sterol per unit volume of the biological sample is lower than the predetermined threshold, it is determined that the diabetes onset risk is high;
(2) When the measured value of ability to uptake sterol per unit volume of the biological sample is equal to or greater than the predetermined threshold, it is determined that the diabetes onset risk is low.

A subject determined to have a high diabetes onset risk may be predicted to have a high possibility of developing diabetes, for example, within 7 years, unless current health condition and lifestyle are improved. On the other hand, a subject determined to have a low diabetes onset risk may be predicted to have a high possibility of avoiding the onset of diabetes if current health condition and lifestyle are maintained. Thus, the determination method of this embodiment makes it possible to provide a medical worker such as a doctor with information that assists determination of diabetes onset risk. Diabetes can be prevented by positively performing interventions for improving lifestyle such as diet and exercise therapy, on subjects determined to have a high diabetes onset risk according to the determination method of this embodiment.

The predetermined threshold may be plural. For example, when a first threshold and a second threshold are used as two predetermined thresholds, subjects can be classified into a high risk group, a medium risk group, and a low risk group. For example, the diabetes onset risk can be determined as follows. In the following example, the first threshold is a value lower than the second threshold:

(1) When the measured value of ability to uptake sterol per unit volume of the biological sample is less than or equal to the first threshold, it is determined that the diabetes onset risk is high;
(2) When the measured value of ability to uptake sterol per unit volume of the biological sample is higher than the first threshold and lower than the second threshold, it is determined that follow-up observation is required;
(3) When the measured value of ability to uptake sterol per unit volume of the biological sample is equal to or greater than the second threshold, it is determined that the diabetes onset risk is low.

When it is determined that follow-up observation is required, it is not appropriate to determine the level of diabetes onset risk, but the diabetes onset risk is not low. That is, the subject is classified into the middle risk group of diabetes onset. Therefore, the subject can be advised to pay attention to health condition and lifestyle.

In the above determination, when the measured value of ability to uptake sterol per unit volume of the biological sample is equal to the first threshold, it has been determined that the diabetes onset risk is high, but it may be determined that follow-up observation is required. In the above determination, when the measured value of ability to uptake sterol per unit volume of the biological sample is equal to the second threshold, it has been determined that the diabetes onset risk is low, but it may be determined that follow-up observation is required.

The predetermined threshold is not particularly limited. The predetermined threshold can be appropriately set. For example, the predetermined threshold may be set empirically by accumulating data of lipoprotein's ability to uptake sterol of non-diabetic subjects. Alternatively, the predetermined threshold may be set as follows. First, biological samples are collected from a plurality of non-diabetic subjects, and lipoprotein's ability to uptake sterol is measured to obtain data of measured values of ability to uptake sterol per unit volume of the biological samples. After lapse of a predetermined period from collection of biological samples, whether or not these subjects develop diabetes is confirmed. The above data are classified into data of a group of subjects who developed diabetes and data of a group of subjects who did not develop diabetes. Then, a value that can most accurately distinguish between the group of subjects who developed diabetes and the group of subjects who did not develop diabetes is determined, and the value is set as a predetermined threshold. In setting the threshold level, it is preferable to consider sensitivity, specificity, positive predictive value, negative predictive value, and the like.

[3. Reagent Kit for Obtaining Information on Diabetes]

The scope of the present disclosure also includes a reagent kit used for the method and determination method of this embodiment. That is, a reagent kit for obtaining information on diabetes (hereinafter, also referred to as a "reagent kit"), including labeled sterol and a capture body that binds to lipoprotein is provided. The details of the labeled sterol and the capture body that binds to lipoprotein are the same as described for the labeled sterol and the first capture body used in the method of this embodiment.

When the labeled sterol is tagged sterol, the reagent kit of this embodiment may further include a capture body that binds to the tag. The reagent kit of this embodiment may further include a labeling substance for labeling the capture body. In this embodiment, the labeling substance may be previously bound to the capture body (the obtained capture body is also referred to as the "labeled capture body"). Thus, the reagent kit of this embodiment may further include a labeled capture body that binds to the tag. When the labeling substance is an enzyme, the reagent kit of this embodiment may further contain a substrate for the enzyme. The details of the capture body that binds to the tag, the labeling substance and the substrate are the same as those described for the second capture body, the second label and the detection of the signal derived from the label.

The reagent kit of this embodiment may include a capture body for measuring the amount of lipoprotein captured. The capture body is not particularly limited as long as it is a substance capable of specifically binding to a part of the surface of lipoprotein. The details of the capture body are similar to those described for the third capture body. The capture body for measurement of the amount of lipoprotein captured may be the same as or different from the capture body that binds to lipoprotein. When these capture bodies are antibodies that bind to the same antigen (for example, ApoAI), each other’s epitopes are preferably different.

The reagent kit of this embodiment may further include a labeling substance for labeling a capture body for measuring the amount of lipoprotein captured. Alternatively, the reagent kit of this embodiment may further include a labeled capture body for measuring the amount of lipoprotein captured. When the labeling substance is an enzyme, the reagent kit of this embodiment may further contain a substrate for the enzyme. The details of the labeling substance and the substrate are the same as those described for the third label and the detection of the signal derived from the label.

It is preferable that the labeled sterol, the various capture bodies, the labeling substance and the substrate be stored in separate containers or individually packaged. The forms of the labeled sterol, the various capture bodies, the labeling substance and the substrate are not particularly limited, and they may be a solid (for example, powder, crystal, freeze-dried product, and the like) or liquid (for example, solution, suspension, emulsion, and the like).

In this embodiment, a container containing a first reagent and a container containing a second reagent may be packed in a box and provided to the user. The box may contain an attached document. The attached document may describe a configuration of the reagent kit, method of use, relationship between the value measured by the reagent kit and the diabetes onset risk, and the like. An example of the reagent kit is shown in the figure. Referring to Fig. 1, 10 denotes a reagent kit, 11 denotes a first container containing labeled sterol, 12 denotes a second container containing a capture body that binds to lipoprotein, 13 denotes a packing box, and 14 denotes an attached document.

The reagent kit of this embodiment may further include a solid phase for immobilizing a capture body that binds to lipoprotein. Referring to Fig. 2, 20 denotes a reagent kit, 21 denotes a first container containing labeled sterol, 22 denotes a second container containing a capture body that binds to lipoprotein, 23 denotes a packing box, 24 denotes an attached document, and 25 indicates a solid phase. In the figure, 25 shows a 96-well microplate as a solid phase, but the present disclosure is not limited to this example.

[4. Determination Apparatus and Computer Program]

The scope of the present disclosure also includes a device and a computer program for performing the method for determining diabetes onset risk of this embodiment. An example of the apparatus for determining diabetes onset risk of this embodiment will be described with reference to the drawings. However, this embodiment is not limited only to the embodiment shown in this example. A determination apparatus 10 shown in Fig. 3 includes a measuring device 20 and a computer system 30 connected to the measuring device 20.

The measuring device is not particularly limited as long as it can detect a signal based on the labeled sterol incorporated into lipoprotein. The measuring device can be appropriately selected according to the type of label of labeled sterol. In the above example, the measuring device 20 is a plate reader that detects a signal based on the labeled sterol incorporated into lipoprotein on the microplate. The signal is optical information such as a fluorescence signal. In this case, when a microplate on which a complex containing lipoprotein incorporating labeled sterol is immobilized is set in the measuring device 20, the measuring device 20 obtains optical information based on the labeled sterol, and the measuring device 20 transmits the obtained optical information to the computer system 30.

As necessary, the amount of lipoprotein captured may be measured by the measuring device. In this case, when the microplate on which a complex of lipoprotein and a labeled capture body that binds to the lipoprotein is

immobilized is set in the measuring device 20, the measuring device 20 obtains optical information based on the labeled capture body, and the measuring device 20 transmits the obtained optical information to the computer system 30. The microplate for measuring the amount of lipoprotein captured and the microplate for measuring ability to uptake sterol of lipoprotein may be the same or different. The label capture body is the same as the third capture body labeled with the third label.

The computer system 30 includes a computer main body 300, an input unit 301, and a display unit 302 that displays specimen information, a determination result, and the like. The computer system 30 receives the optical information from the measuring device 20. Then, the processor of the computer system 30 executes a program for determining diabetes onset risk, based on the optical information. As shown in Fig. 3, the computer system 30 may be equipment separate from the measuring device 20, or may be equipment including the measuring device 20. In the latter case, the computer system 30 may itself be the determination apparatus 10.

Referring to Fig. 4, the computer main body 300 includes a central processing unit (CPU) 310, a read only memory (ROM) 311, a random access memory (RAM) 312, a hard disk 313, an input/output interface 314, a reading device 315, a communication interface 316, and an image output interface 317. The CPU 310, the ROM 311, the RAM 312, the hard disk 313, the input/output interface 314, the reading device 315, the communication interface 316 and the image output interface 317 are data-communicably connected by a bus 318. The measuring device 20 is communicably connected to the computer system 30 via the communication interface 316.

The CPU 310 can execute a program stored in the ROM 311 or the hard disk 313 and a program loaded in the RAM 312. The CPU 310 obtains a measured value of ability to uptake sterol per unit volume of the biological sample, based on the optical information obtained from the measuring device 20. Details of the measured value of ability to uptake sterol per unit volume of the biological sample and the calculation thereof are the same as those described for the method of this embodiment. When the diluted biological sample (or lipoprotein fraction) is used, the measured value of ability to uptake sterol per unit volume of the biological sample is calculated according to the equation (1) stored in the ROM 311 or the hard disk 313. Then, the CPU 310 determines diabetes onset risk based on the obtained measured value and the predetermined threshold stored in the ROM 311 or the hard disk 313. The CPU 310 outputs the determination result and displays the determination result on the display unit 302.

The ROM 311 includes a mask ROM, PROM, EPROM, EEPROM, and the like. In the ROM 311, a computer program executed by the CPU 310 and data used for executing the computer program are recorded.

The RAM 312 includes SRAM, DRAM, and the like. The RAM 312 is used for reading the program recorded in the ROM 311 and the hard disk 313. The RAM 312 is also used as a work area of the CPU 310 when these programs are executed.

The hard disk 313 has installed therein an operating system to be executed by the CPU 310, a computer program such as an application program (program for determining diabetes onset risk), and data used for executing the computer program.

The reading device 315 includes a flexible disk drive, a CD-ROM drive, a DVD-ROM drive, and the like. The reading device 315 can read a program or data recorded on a portable recording medium 40.

The input/output interface 314 includes, for example, a serial interface such as USB, IEEE1394 and RS-232C, a parallel interface such as SCSI, IDE and IEEE1284, and an analog interface including a D/A converter, an A/D converter and the like. The input unit 301 such as a keyboard and a mouse is connected to the input/output interface 314. An operator can input various commands to the computer main body 300 through the input unit 301.

The communication interface 316 is, for example, an Ethernet (registered trademark) interface or the like. The computer main body 300 can also transmit print data to a printer or the like through the communication interface 316.

The image output interface 317 is connected to the display unit 302 including an LCD, a CRT, and the like. As a result, the display unit 302 can output a video signal corresponding to the image data coming from the CPU 310. The display unit 302 displays an image (screen) according to the input video signal.

Referring to Fig. 5, a processing procedure for determining diabetes onset risk executed by the determination apparatus 10 will be described. Here, a case of determining diabetes onset risk based on a fluorescence signal generated from a fluorescently labeled sterol incorporated into lipoprotein on the microplate will be described as an example. However, this embodiment is not limited to this example.

In step S101, the CPU 310 obtains optical information (fluorescent signal) from the measuring device 20, and the CPU 310 stores the optical information in the hard disk 313. In step S102, the CPU 310 calculates a measured value of ability to uptake sterol per unit volume of the biological sample from the obtained optical information, and the CPU 310 stores the measured value in the hard disk 313. In step S103, the CPU 310 compares the calculated measured value with the predetermined threshold stored in the hard disk 313. When the measured value is less than or equal to the predetermined threshold, the process proceeds to step S104, and a determination result indicating that the diabetes onset risk of the subject is high is stored in the hard disk 313. When the measured value is higher than the predetermined threshold, the process proceeds to step S105, and a determination result indicating that the diabetes onset risk of the subject is low is stored in the hard disk 313. In step S106, the CPU 310 outputs the determination result, and the CPU

310 displays the determination result on the display unit 302, or the CPU 310 makes a printer print out the determination result. Accordingly, it is possible to provide doctors and the like with information to assist the determination of diabetes onset risk.

Referring to Fig. 6, a processing procedure for determining diabetes onset risk using two predetermined thresholds will be described. Here, a case of determining diabetes onset risk based on a fluorescence signal generated from a fluorescently labeled sterol incorporated into lipoprotein on the microplate will be described as an example. However, this embodiment is not limited to this example.

Details of steps S201, S202 and S208 are similar to those described for the steps S101, S102 and S106, respectively. In step S203, the CPU 310 compares the calculated measured value with the first threshold stored in the hard disk 313. When the measured value is less than or equal to the first threshold, the process proceeds to step S204, and a determination result indicating that the diabetes onset risk of the subject is high is stored in the hard disk 313. When the measured value is higher than the first threshold, the process proceeds to step S205. In step S205, the CPU 310 compares the calculated measured value with the second threshold stored in the hard disk 313. When the measured value is equal to or greater than the second threshold, the process proceeds to step S206, and a determination result indicating that the diabetes onset risk of the subject is low is stored in the hard disk 313. When the measured value is lower than the second threshold, the process proceeds to step S207, and a determination result indicating that follow-up observation of the subject is required is stored in the hard disk 313.

Hereinafter, the present disclosure will be described in more detail by examples, but the present disclosure is not limited to these examples.

## EXAMPLES

### Example 1

Using blood samples from a Hisayama study (an epidemiological study conducted for residents in Hisayama-cho, Kasuya-gun, Fukuoka Prefecture), relationship between HDL's ability to uptake cholesterol and onset of diabetes was examined.

(1) Target population

Information on a target population is shown in Table 1. Excluded subjects were those who had developed diabetes, those who did not have a glucose tolerance test or those who had a history of cardiovascular disease by the time of enrollment. Those who did not have a glucose tolerance test were excluded because it was unclear whether or not they had developed diabetes. Those who had a history of cardiovascular disease refer to those who have experienced myocardial infarction, cerebral infarction or coronary angioplasty by the time of enrollment.

[Table 1]

| | |
|---|---|
| Year of enrollment | 2002 |
| Age | 40 to 79 |
| Number of subjects | 2091 cases |
| Excluded subjects | Persons who had developed diabetes, persons who did not have glucose tolerance test, persons who had history of cardiovascular disease |
| Observation period | 7 years |

(2) Measurement of HDL's ability to uptake cholesterol

(2.1) Biological sample

Cryopreserved serum of each subject was used as a biological sample. Thawed serum (0.1 mL) was mixed with an equal amount of 22% polyethylene glycol 4000 (Nacalai Tesque, Inc.) to obtain a suspension. The resulting suspension was allowed to stand at room temperature for 20 minutes and then centrifuged at 3000 rpm at room temperature for 15 minutes. The obtained supernatant was used as an HDL fraction.

(2.2) Formation of complex of HDL and anti-ApoAI antibody on solid phase (i) Preparation of measurement plate (Immobilization of anti-ApoAI antibody to solid phase)

200 μl each of 50 mM Tris-HCl (pH 7.5) was added to each well of a 96-well microplate (black plate H for fluorescence measurement, manufactured by Sumitomo Bakelite Co., Ltd.) as a solid phase for washing. This washing operation was performed twice in total. To each well was added 100 μl each of a solution of anti-ApoAI antibody (clone 1C5, Cat. No. MONO5030, SANBIO B.V.) diluted to a concentration of 10 μg/ml with 50 mM Tris-HCl (pH 7.5), and the well was allowed to stand at 4°C over night. The antibody solution was removed, and 200 μl each of PBS was added to each well for washing. This washing operation was performed three times in total. 200 μl each of 2% BSA/PBS was added to each well, and the microplate was shaken at 600 rpm at 25°C for 2 hours.

(ii) Preparation of measurement sample (Contact between HDL and labeled cholesterol)

A portion was taken from the serum of the subject, and ApoAI concentration was measured using a kit for ApoAI measurement (N-assay TIA ApoAI-H, Nittobo Medical Co., Ltd.). Specific operations for measuring the concentration were performed according to a manual attached to the kit. Since the HDL fraction was prepared from serum diluted 2-fold with PEG, the ApoAI concentration of the HDL fraction was set to 1/2 of the ApoAI concentration of serum obtained by the measurement. Each HDL fraction was diluted so that the ApoAI concentration in the HDL fraction was 1.25 to 2 μg/ml, to prepare an HDL fraction-containing diluent. For dilution of the HDL fraction, PBS (hereinafter also referred to as "buffer") containing 2% BSA and 2 mM liposome (manufactured by NIPPON FINE CHEMICAL CO., LTD.) was used. The buffer was used as a control specimen containing no HDL fraction (ApoAI concentration 0 μg/ml). The composition of the liposome contained in the buffer was 2 mM dimyristoyl phosphatidyl glycerol (DMPG), 2 mM cholesterol and 4 mM hydrogenated soybean phosphatidyl choline (HSPC). PBS was prepared by dissolving Phosphate buffered saline tablet (Sigma-Aldrich) in water.

After 0.5 mM BODIPY-added cholesterol (TopFluor Cholesterol, Avanti Polar Lipids, Inc.) was added to the buffer to a final concentration of 5 μM, the HDL fraction-containing diluent was further added at 1/50 of the total volume. The resulting mixture was shaken at 800 rpm at 37°C for 2 hours. To the resulting mixed solution was added an oxidizing agent (8.8 M hydrogen peroxide, 1.76 mM sodium nitrite and 0.86 mM diethylene triamine pentaacetic acid (DTPA)). The final concentration of each component in the solution to which the oxidizing agent was added was 1 M of hydrogen peroxide, 200 μM of sodium nitrite, and 100 μM of DTPA. These solutions were shaken at 800 rpm at 37°C for 1 hour to obtain a measurement sample containing HDL incorporated with BODIPY-added cholesterol.

(iii) Formation of complex of HDL incorporating cholesterol and anti-ApoAI antibody

The BSA solution was removed from the plate on which the anti-ApoAI antibody was immobilized, and 100 μl each of each measurement sample was added to the wells. Then, the plate was shaken at 600 rpm at 25°C for 1 hour to form a complex of HDL and the anti-ApoAI antibody on the plate.

(2.3) Measurement of HDL's ability to uptake cholesterol

The intensity of fluorescence derived from BODIPY-added cholesterol incorporated into HDL was measured as follows. 100 μl each of 10 mM cyclodextrin/PBS was added to each well of the plate on which the complex of HDL and the anti-ApoAI antibody was captured, and the plate was shaken at 600 rpm at 25°C for 1 hour. Then, the fluorescence intensity was measured by a fluorescence plate reader (Infinite (registered trademark) 200 Pro, manufactured by TECAN) (excitation light 485 nm/fluorescence 535 nm).

(2.4) Measurement of amount of HDL captured

The amount of HDL captured was measured, based on the concentration of ApoAI contained in HDL in the complex on the plate. Specifically, it was measured as follows. The cyclodextrin solution was removed from the plate after the measurement of (2.3) above, and each well was washed three times with PBS. Goat anti-ApoAI serum of ApoAI measurement kit (N-assay TIA ApoAI-H, Nittobo Medical Co., Ltd.) was diluted 1,000-fold with a blocking buffer (StartingBlock, Thermo Scientific), and 100 μl each of the obtained diluent was added to each well. The plate was shaken at 600 rpm at 25°C for 1 hour, then the diluent was removed and each well was washed three times with PBS. HRP-labeled rabbit anti-goat IgG polyclonal antibody (P0449, Dako) was diluted 1,000-fold with a blocking buffer (StartingBlock, Thermo Scientific), and 100 μl each of the obtained diluent was added to each well. The plate was shaken at 600 rpm at 25°C for 1 hour, then the diluent was removed, and each well was washed five times with PBS. 100 μl each of a chemiluminescent substrate solution (SuperSignal ELISA Pico, 37069, Thermo Scientific) was added to each well. After

shaking the plate at 600 rpm at 25°C for 2 minutes, the amount of luminescence was measured with a microplate reader (Infinite (registered trademark) F200 Pro, manufactured by TECAN).

(3) Analysis

As described above, in Example 1, the HDL fraction prepared from a predetermined amount of serum was diluted so that the concentration of ApoAI was constant, and was used to measure ability to uptake cholesterol. Therefore, the measured value obtained in (2.3) above was converted to a measured value of ability to uptake labeled cholesterol per unit volume of serum by the following formula (2). In the formula, the "measured value of ability to uptake labeled cholesterol" is the measured value obtained in (2.3) above, the "amount of HDL captured" is the measured value obtained in (2.4) above, and the "ApoA1 concentration in serum" was the measured value obtained in (2.2) (ii) above.

$$\text{(Measured value of ability to uptake labeled cholesterol per unit volume of serum)} = [\text{(Measured value of ability to uptake labeled cholesterol)}/\text{(Amount of HDL captured)}] \times \text{(ApoA1 concentration in serum)} \ldots \quad (2)$$

(4) Results

Among 2091 subjects, 219 cases developed diabetes during the observation period. Hazard ratios were calculated using a Cox proportional hazards model, and were adjusted for potential confounders (age, gender, family history of diabetes, systolic blood pressure, antihypertensive drugs, total cholesterol level, triglyceride level, lipid-improving agents, obesity/overweight, smoking, drinking, and exercise). The subjects were classified into four groups based on the value of ability to uptake cholesterol per unit volume of serum. The ability to uptake cholesterol and hazard ratio of each group are shown in Table 2.

[Table 2]

| | Ability to uptake cholesterol per unit volume of serum | N | Hazard ratio (95% confidence interval) | p-Value | p-value for trend |
|---|---|---|---|---|---|
| Q1 | ≤ 44.7 | 525 | 1.00 (Reference) | | 0.06 |
| Q2 | 44.8-51.6 | 515 | 0.60 (0.40, 0.92) | 0.02 | |
| Q3 | 51.7-59.4 | 528 | 0.88 (0.60, 1.30) | 0.52 | |
| Q4 | ≥ 59.5 | 522 | 0.57 (0.36, 090) | 0.01 | |

The hazard ratio significantly decreased with an increase in HDL's ability to uptake cholesterol. The hazard ratio of a Q4 (fourth quartile) group was 0.57 times that of a Q1 (first quartile) group. That is, it was shown that when the measured value of ability to uptake cholesterol per unit volume of the biological sample is higher than a predetermined value, the diabetes onset risk is low. It was shown that when the measured value of ability to uptake cholesterol per unit volume of the biological sample is lower than the predetermined value, the diabetes onset risk is high. Therefore, it was shown that the diabetes onset risk can be determined by evaluating the ability to uptake cholesterol per unit volume of the biological sample per ApoA1 in subjects who do not have a history of cardiovascular disease and are not diabetic.

Example 2

Relationship between the HDL's ability to uptake cholesterol and the diabetes onset risk was examined in the same manner as in Example 1 except that subjects (66 cases) who had a history of cardiovascular disease excluded in Example 1 were added. The results are shown in Table 3.

[Table 3]

| | Ability to uptake cholesterol per unit volume of serum | N | Hazard ratio (95% confidence interval) | p-Value | p-value for trend |
|---|---|---|---|---|---|
| Q1 | ≤ 44.6 | 535 | 1.00 (Reference) | | 0.07 |
| Q2 | 44.7-51.6 | 542 | 0.61 (0.40, 0.91) | 0.02 | |
| Q3 | 51.7-59.4 | 540 | 0.86 (0.59, 1.26) | 0.44 | |
| Q4 | ≥ 59.5 | 539 | 0.60 (0.38, 0.92) | 0.02 | |

The hazard ratio significantly decreased with an increase in HDL's ability to uptake cholesterol. The hazard ratio of a Q4 (fourth quartile) group was 0.60 times that of a Q1 (first quartile) group. That is, it was shown that when the measured value of ability to uptake cholesterol per unit volume of the biological sample is higher than a predetermined value, the diabetes onset risk is low. It was shown that when the measured value of ability to uptake cholesterol per unit volume of the biological sample is lower than the predetermined value, the diabetes onset risk is high. Therefore, it was shown that the diabetes onset risk can be determined by evaluating the ability to uptake cholesterol per unit volume of the biological sample per ApoA1 in non-diabetic subjects.

## Claims

**1.** A method for obtaining information on subject's diabetes, comprising measuring lipoprotein's ability to uptake sterol in a biological sample of a subject, wherein a measured value of ability to uptake sterol per unit volume of the biological sample is an indicator of diabetes onset risk.

**2.** The method according to claim 1, wherein the subject is a subject without a history of cardiovascular disease.

**3.** The method according to claim 1 or 2, wherein the biological sample is a blood sample.

**4.** The method according to any one of claims 1 to 3, wherein the lipoprotein is high-density lipoprotein.

**5.** The method according to any one of claims 1 to 4, wherein the measurement step is performed in a cell-free system.

**6.** The method according to any one of claims 1 to 5, wherein the measurement step comprises the steps of:

preparing lipoprotein incorporating labeled sterol by contacting the lipoprotein in the biological sample with the labeled sterol; and
measuring ability to uptake the labeled sterol, based on the label of the labeled sterol incorporated into the lipoprotein.

**7.** The method according to claim 6, wherein the labeled sterol is the labeled cholesterol.

**8.** The method according to claim 7, wherein the labeled cholesterol is tagged cholesterol represented by the following formula (I):

[Chemical Formula 1]

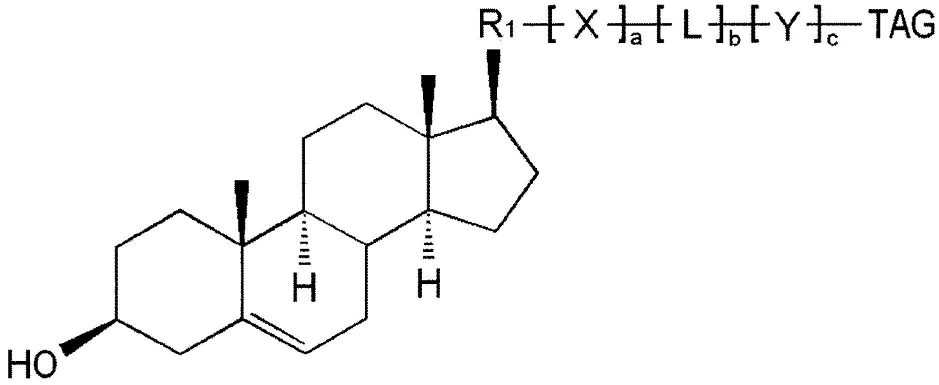

(I)

wherein $R_1$ is an alkylene group having 1 to 6 carbon atoms which may have a methyl group,
X and Y are identical or different, and are represented by $-R_2-NH-$, $-NH-R_2-$, $-R_2-(C=O)-NH-$, $-(C=O)-NH-R_2-$, $-R_2-NH-(C=O)-$, $-NH-(C=O)-R_2-$, $-R_2-(C=O)-$, $-(C=O)-R_2-$, - $R_2-(C=O)-O-$, $-(C=O)-O-R_2-$, $-R_2-O-(C=O)-$, $-O-(C=O)-R_2-$, $-R_2-(C=S)-NH-$, $-(C=S)-NH-R_2-$, $-R_2-NH-(C=S)-$, $-NH-(C=S)-R_2-$, $-R_2-O-$, $-O-R_2-$, $-R_2-S-$, or $-S-R_2-$, and each $R_2$ is independently an atomic bonding, an alkylene group having 1 to 10 carbon atoms which may have a substituent, or an arylene group or heteroarylene group having 6 to 12 carbon atoms which may have a substituent, or a cycloalkylene group or herocycloalkylene group having 3 to 8 carbon atoms which may have a substituent;
L is represented by $-(CH_2)_d-[R_3-(CH_2)_e]_f-$ or $-[(CH_2)_e-R_3]_f-(CH_2)d-$, and $R_3$ is an oxygen atom, a sulfur atom, -NH-, -NH-(C=O)- or -(C=O)-NH-;
TAG is a tag;
a and c are identical or different and are an integer of 0 to 6;
b is 0 or 1;
d and e are identical or different and are an integer of 0 to 12; and
f is an integer of 0 to 24.

**9.** The method according to any one of claims 6 to 8, wherein the measurement step comprises the step of contacting the lipoprotein incorporating labeled sterol with a capture body that binds to the lipoprotein to form a complex containing the lipoprotein incorporating labeled sterol and the first capture body.

**10.** The method according to claim 9, wherein the capture body is immobilized on a solid phase, and the lipoprotein is immobilized on the solid phase.

**11.** The method according to any one of claims 6 to 10, wherein the measurement step is performed by detecting a signal derived from the labeled sterol incorporated into the lipoprotein.

**12.** The method according to any one of claims 1 to 11, wherein it is determined that the diabetes onset risk is high when the measured value of ability to uptake sterol per unit volume of the biological sample is less than or equal to a predetermined threshold, and that the diabetes onset risk is low when the measured value of ability to uptake sterol per unit volume of the biological sample is higher than the predetermined threshold.

**13.** Use of a reagent kit in a method for obtaining information on diabetes, comprising labeled sterol and a capture body that binds to lipoprotein.

**14.** An apparatus for determining diabetes onset risk, comprising a computer comprising a processor and a memory under control of the processor, wherein the memory is recorded with a computer program for causing the computer to execute the steps of:

obtaining a measured value of lipoprotein's ability to uptake sterol in a biological sample of a subject;
determining diabetes onset risk, based on the measured value of ability to uptake sterol per unit volume of the biological sample; and
outputting a result of the determination.

**15.** A computer program for determining diabetes onset risk, which is recorded on a computer readable medium, and causes the computer to execute the following steps:

obtaining a measured value of lipoprotein's ability to uptake sterol in a biological sample of a subject;
determining diabetes onset risk, based on the measured value of ability to uptake sterol per unit volume of the biological sample; and
outputting a result of the determination.

## Patentansprüche

**1.** Verfahren zum Erhalten von Informationen über den Diabetes eines Subjekts, umfassend das Messen der Fähigkeit von Lipoprotein zur Aufnahme von Sterin in einer biologischen Probe eines Subjekts, wobei ein gemessener Wert der Fähigkeit zur Aufnahme von Sterin pro Einheitsvolumen der biologischen Probe ein Indikator für ein Risiko des Einsetzens von Diabetes ist.

**2.** Verfahren gemäß Anspruch 1, wobei das Subjekt ein Subjekt ohne Vorgeschichte von kardiovaskulärer Erkrankung ist.

**3.** Verfahren gemäß Anspruch 1 oder 2, wobei die biologische Probe eine Blutprobe ist.

**4.** Verfahren gemäß irgendeinem der Ansprüche 1 bis 3, wobei das Lipoprotein Lipoprotein hoher Dichte ist.

**5.** Verfahren gemäß irgendeinem der Ansprüche 1 bis 4, wobei der Messschritt in einem zellfreien System durchgeführt wird.

**6.** Verfahren gemäß irgendeinem der Ansprüche 1 bis 5, wobei der Messschritt die folgenden Schritte umfasst:

Herstellen von Lipoprotein, welches markiertes Sterin inkorporiert, durch Inkontaktbringen des Lipoproteins in der biologischen Probe mit dem markierten Sterin; und
Messen der Fähigkeit zur Aufnahme des markierten Sterins auf Grundlage der Markierung des markierten Sterins, das in dem Lipoprotein inkorporiert ist.

**7.** Verfahren gemäß Anspruch 6, wobei das markierte Sterin das markierte Cholesterin ist.

**8.** Verfahren gemäß Anspruch 7, wobei das markierte Cholesterin getaggtes Cholesterin ist, repräsentiert durch die folgende Formel (I):

[Chemische Formel 1]

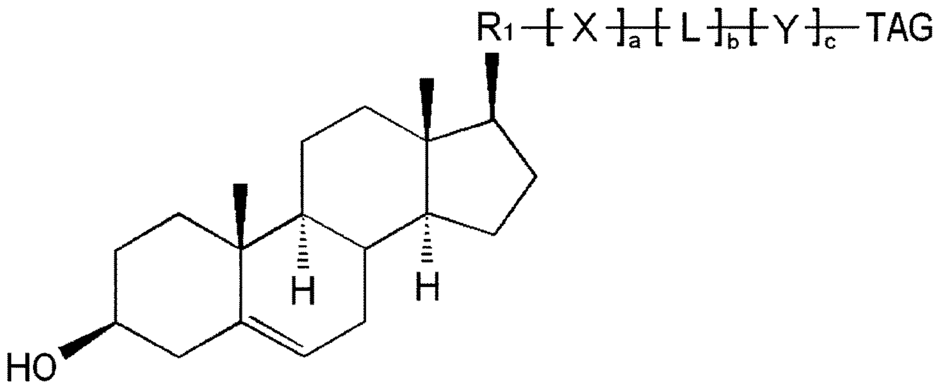

(I)

worin $R_1$ eine Alkylengruppe mit 1 bis 6 Kohlenstoffatomen ist, die eine Methylgruppe aufweisen können,
X und Y identisch oder verschieden sind und repräsentiert sind durch $-R_2$-NH-, -NH-$R_2$-, $-R_2$-(C=O)-NH-, - (C=O) -NH-$R_2$-, $-R_2$-NH- (C=O)-, -NH- (C=O) -$R_2$-, $-R_2$- (C=O) -, - (C=O) -$R_2$-, $-R_2$- (C=O) -O-, - (C=O) -O-$R_2$-, $-R_2$-O- (C=O) -, -O-(C=O)-$R_2$-, $-R_2$-(C=S)-NH-, -(C=S)-NH-$R_2$-, $-R_2$-NH-(C=S)-, -NH-(C=S)-$R_2$-, $-R_2$-O-, -O-$R_2$-, $-R_2$-S- oder -S-$R_2$-, und jeder $R_2$ unabhängig eine atomare Bindung, eine Alkylengruppe mit 1 bis 10 Kohlenstoffatomen,

die einen Substituenten aufweisen können, oder eine Arylengruppe oder Heteroarylengruppe mit 6 bis 12 Kohlenstoffatomen, die einen Substituenten aufweisen können, oder eine Cycloalkylengruppe oder Heterocycloalkylengruppe mit 3 bis 8 Kohlenstoffatomen, die einen Substituenten aufweisen können, ist;
L repräsentiert ist durch - $(CH_2)_d$-$[R_3$-$(CH_2)_e]_f$- oder - $[(CH_2)_e$-$R_3]_f$-$(CH_2)_d$-, und $R_3$ ein Sauerstoffatom, ein Schwefelatom, -NH-, -NH-(C=O)- oder -(C=O)-NH- ist;
TAG ein Tag ist;
a und c identisch oder verschieden sind und eine ganze Zahl von 0 bis 6 sind;
b 0 oder 1 ist;
d und e identisch oder verschieden sind und eine ganze Zahl von 0 bis 12 sind; und
f eine ganze Zahl von 0 bis 24 ist.

**9.** Verfahren gemäß irgendeinem der Ansprüche 6 bis 8, wobei der Messschritt den Schritt des Inkontaktbringens des Lipoproteins, welches markiertes Sterin inkorporiert, mit einem Einfangkörper, welcher an das Lipoprotein bindet, umfasst, um einen Komplex zu bilden, der das markiertes Sterin inkorporierende Lipoprotein und den ersten Einfangkörper enthält.

**10.** Verfahren gemäß Anspruch 9, wobei der Einfangkörper auf einer festen Phase immobilisiert ist und das Lipoprotein auf der festen Phase immobilisiert ist.

**11.** Verfahren gemäß irgendeinem der Ansprüche 6 bis 10, wobei der Messschritt durch Detektieren eines Signals durchgeführt wird, welches von dem in das Lipoprotein inkorporierten markierten Sterin abgeleitet ist.

**12.** Verfahren gemäß irgendeinem der Ansprüche 1 bis 11, wobei das Risiko des Einsetzens von Diabetes als hoch bestimmt wird, wenn der gemessene Wert der Fähigkeit zur Aufnahme von Sterin pro Einheitsvolumen der biologischen Probe kleiner als ein oder gleich einem vorbestimmten Schwellwert ist, und das Risiko des Einsetzens von Diabetes als gering bestimmt wird, wenn der gemessene Wert der Fähigkeit zur Aufnahme von Sterin pro Einheitsvolumen der biologischen Probe höher ist als der vorbestimmte Schwellwert.

**13.** Verwendung eines Reagenskits in einem Verfahren zum Erhalten von Informationen über Diabetes, umfassend markiertes Sterin und einen Einfangkörper, der an Lipoprotein bindet.

**14.** Vorrichtung zum Bestimmen des Risikos des Einsetzens von Diabetes, umfassend einen Computer, umfassend einen Prozessor und einen der Kontrolle des Prozessors unterstehenden Speicher, wobei der Speicher mit einem Computerprogramm aufgezeichnet wird, welches den Computer dazu bringt, die folgenden Schritte auszuführen:

Erhalten eines gemessenen Werts der Fähigkeit von Lipoprotein zur Aufnahme von Sterin in einer biologischen Probe eines Subjekts;
Bestimmen des Risikos des Einsetzens von Diabetes auf Grundlage des gemessenen Werts der Fähigkeit zur Aufnahme von Sterin pro Einheitsvolumen der biologischen Probe; und
Ausgeben eines Ergebnisses der Bestimmung.

**15.** Computerprogramm zum Bestimmen des Risikos des Einsetzens von Diabetes, welches auf einem computerlesbaren Medium aufgezeichnet ist und den Computer dazu bringt, die folgenden Schritte auszuführen:

Erhalten eines gemessenen Werts der Fähigkeit von Lipoprotein zur Aufnahme von Sterin in einer biologischen Probe eines Subjekts;
Bestimmen des Risikos des Einsetzens von Diabetes auf Grundlage des gemessenen Werts der Fähigkeit zur Aufnahme von Sterin pro Einheitsvolumen der biologischen Probe; und
Ausgeben eines Ergebnisses der Bestimmung.

## Revendications

**1.** Procédé d'obtention d'informations sur un diabète d'un sujet, comprenant la mesure de la capacité d'une lipoprotéine à absorber un stérol dans un échantillon biologique d'un sujet, dans lequel une valeur mesurée de la capacité d'absorption d'un stérol par unité de volume de l'échantillon biologique est un indicateur de risque d'apparition d'un diabète.

**2.** Procédé selon la revendication 1, dans lequel le sujet est un sujet sans antécédent de maladie cardiovasculaire.

**3.** Procédé selon la revendication 1 ou 2, dans lequel l'échantillon biologique est un échantillon de sang.

**4.** Procédé selon l'une quelconque des revendications 1 à 3, dans lequel la lipoprotéine est une lipoprotéine haute densité.

**5.** Procédé selon l'une quelconque des revendications 1 à 4, dans lequel l'étape de mesure est effectuée dans un système acellulaire.

**6.** Procédé selon l'une quelconque des revendications 1 à 5, dans lequel l'étape de mesure comprend les étapes consistant à :

préparer une lipoprotéine incorporant un stérol marqué en mettant en contact la lipoprotéine de l'échantillon biologique avec le stérol marqué ; et
mesurer la capacité d'absorption du stérol marqué, sur la base du marqueur du stérol marqué incorporé dans la lipoprotéine.

**7.** Procédé selon la revendication 6, dans lequel le stérol marqué est le cholestérol marqué.

**8.** Procédé selon la revendication 7, dans lequel le cholestérol marqué est un cholestérol marqué représenté par la formule (I) suivante :

[Formule chimique 1]

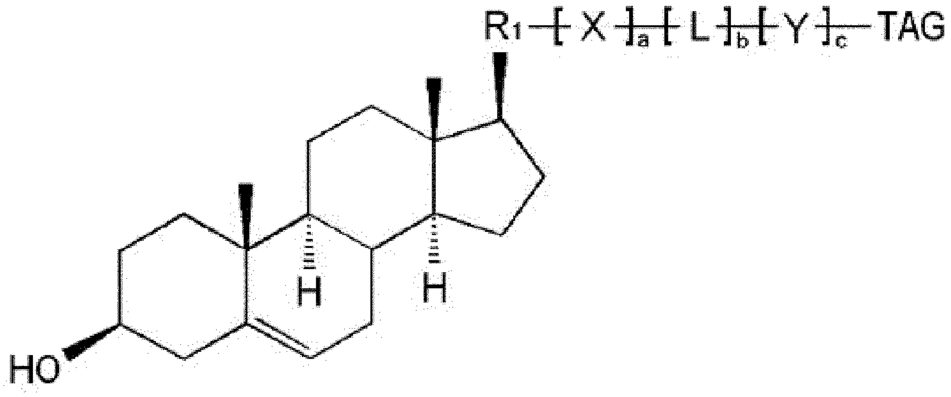

(I)

dans lequel $R_1$ est un groupe alkylène présentant 1 à 6 atomes de carbone qui peut présenter un groupe méthyle,
X et Y sont identiques ou différents, et sont représentés par $-R_2$-NH-, -NH-$R_2$-, $-R_2$-(C=O)-NH-, -(C=O)-NH-$R_2$-, $-R_2$-NH-(C=O)-, -NH-(C=O)-$R_2$-, $-R_2$-(C=O)-, -(C=O)-$R_2$-, $-R_2$-(C=O)-O-, -(C=O)-O-$R_2$-, $-R_2$-O-(C=O)-, -O-(C=O)-$R_2$-, $-R_2$-(C=S)-NH-, -(C=S)-NH-$R_2$-, $-R_2$-NH-(C=S)-, - NH-(C=S)-$R_2$-, $-R_2$-O-, -O-$R_2$-, $-R_2$-S-, ou-S-$R_2$-, et chaque $R_2$ est indépendamment une liaison atomique, un groupe alkylène présentant 1 à 10 atomes de carbone qui peut présenter un substituant, ou un groupe arylène ou un groupe hétéroarylène présentant 6 à 12 atomes de carbone qui peut présenter un substituant, ou un groupe cycloalkylène ou un groupe hétérocycloalkylène présentant 3 à 8 atomes de carbone qui peut présenter un substituant ;
L est représenté par $-(CH_2)_d-[R_3-(CH_2)_e]_f-$ ou $-[(CH_2)_e-R_3]_f-(CH_2)_d-$, et $R_3$ est un atome d'oxygène, un atome de soufre, -NH-, -NH-(C=O)- ou -(C=O)-NH-;
TAG est un marqueur;
a et c sont identiques ou différents et sont un nombre entier de 0 à 6 ;
b est 0 ou 1 ;
d et e sont identiques ou différents et sont un nombre entier de 0 à 12 ; et
f est un nombre entier de 0 à 24.

**9.** Procédé selon l'une quelconque des revendications 6 à 8, dans lequel l'étape de mesure comprend l'étape de mise en contact de la lipoprotéine incorporant un stérol marqué avec un corps de capture qui se lie à la lipoprotéine pour former un complexe contenant la lipoprotéine incorporant un stérol marqué et le premier corps de capture.

10. Procédé selon la revendication 9, dans lequel le corps de capture est immobilisé sur une phase solide, et la lipoprotéine est immobilisée sur la phase solide.

11. Procédé selon l'une quelconque des revendications 6 à 10, dans lequel l'étape de mesure est effectuée en détectant un signal dérivé du stérol marqué incorporé dans la lipoprotéine.

12. Procédé selon l'une quelconque des revendications 1 à 11, dans lequel il est déterminé que le risque d'apparition d'un diabète est élevé quand la valeur mesurée de la capacité d'absorption d'un stérol par unité de volume de l'échantillon biologique est inférieure ou égale à un seuil prédéterminé, et que le risque d'apparition d'un diabète est faible quand la valeur mesurée de la capacité d'absorption d'un stérol par unité de volume de l'échantillon biologique est supérieure au seuil prédéterminé.

13. Utilisation d'un kit de réactifs dans un procédé d'obtention d'informations sur un diabète, comprenant un stérol marqué et un corps de capture qui se lie à une lipoprotéine.

14. Appareil pour déterminer un risque d'apparition d'un diabète, comprenant un ordinateur comprenant un processeur et une mémoire sous le contrôle du processeur, dans lequel un programme informatique est enregistré sur la mémoire pour amener l'ordinateur à exécuter les étapes consistant à :

obtenir une valeur mesurée de la capacité d'une lipoprotéine à absorber un stérol dans un échantillon biologique d'un sujet ;
déterminer le risque d'apparition d'un diabète, sur la base de la valeur mesurée de la capacité d'absorption d'un stérol par unité de volume de l'échantillon biologique ; et
sortir un résultat de la détermination.

15. Programme informatique pour déterminer un risque d'apparition d'un diabète, qui est enregistré sur un support lisible par ordinateur, et amène l'ordinateur à exécuter les étapes suivantes consistant à :

obtenir une valeur mesurée de la capacité d'une lipoprotéine à absorber un stérol dans un échantillon biologique d'un sujet ;
déterminer le risque d'apparition d'un diabète, sur la base de la valeur mesurée de la capacité d'absorption d'un stérol par unité de volume de l'échantillon biologique ; et
sortir un résultat de la détermination.

FIG. 1

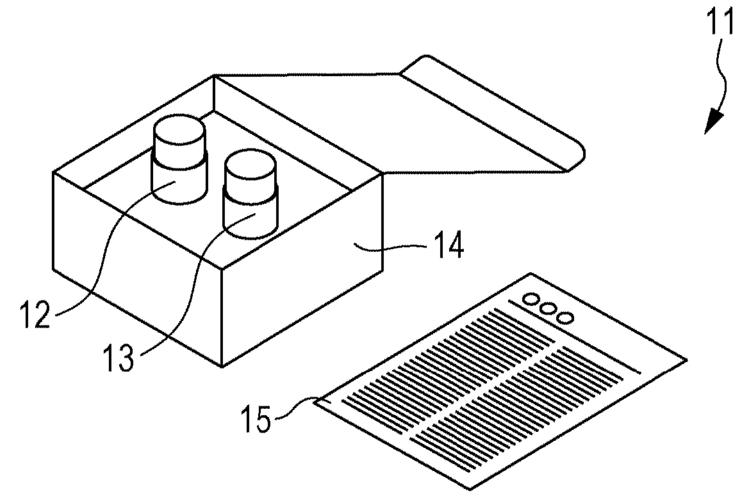


FIG. 2

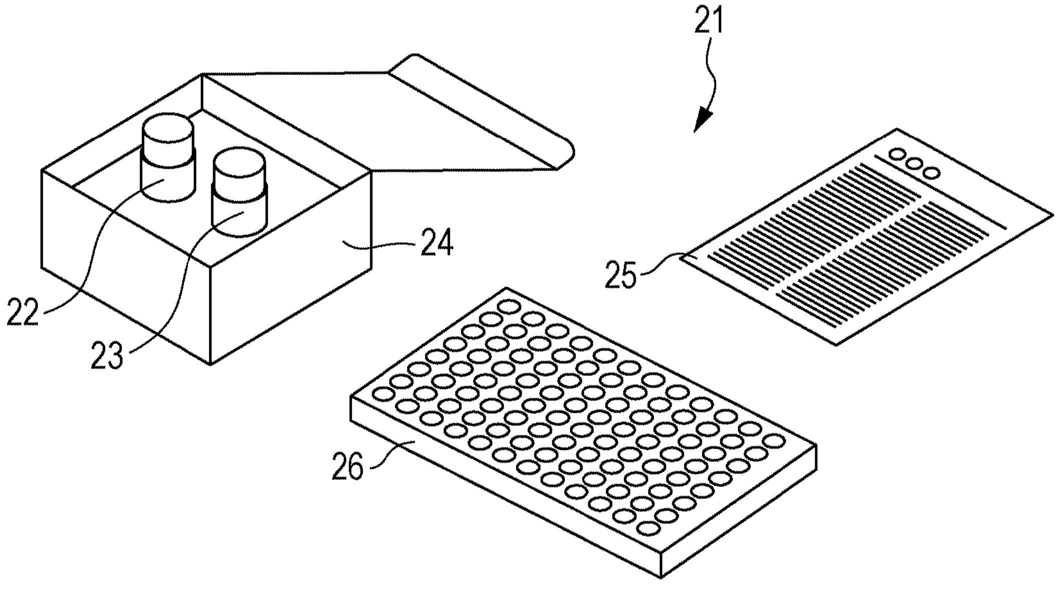

FIG. 3

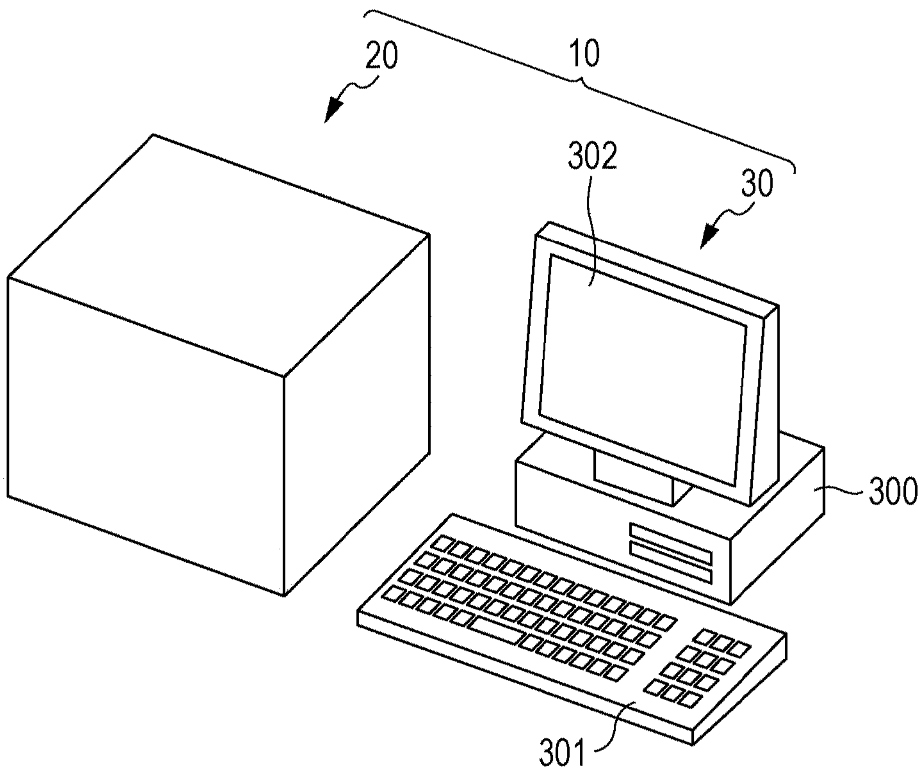

FIG. 4

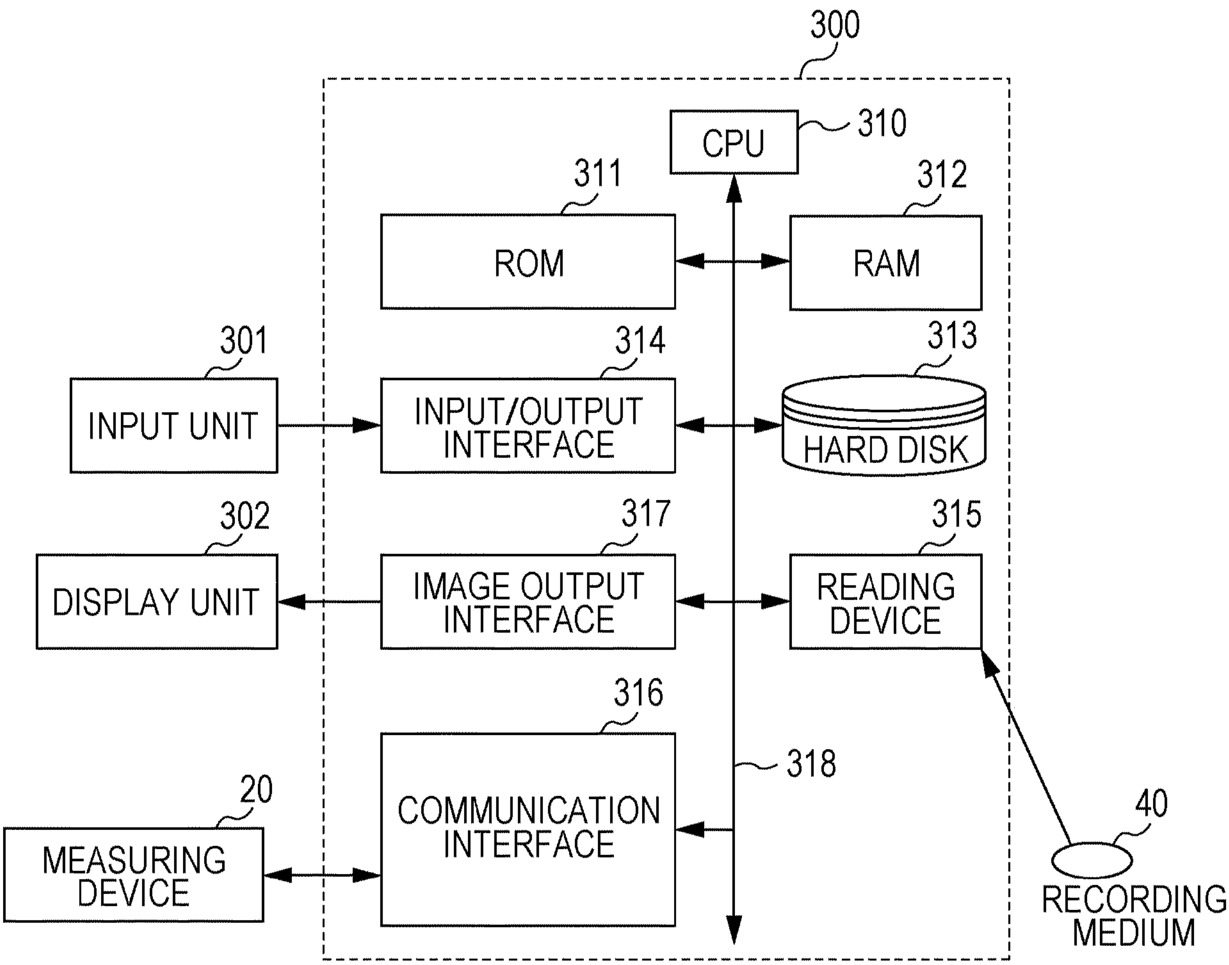
300
CPU
310
311
312
ROM
RAM
301
314
313
INPUT UNIT
INPUT/OUTPUT INTERFACE
HARD DISK
302
317
315
DISPLAY UNIT
IMAGE OUTPUT INTERFACE
READING DEVICE
316
318
20
40
MEASURING DEVICE
COMMUNICATION INTERFACE
RECORDING MEDIUM

FIG. 5

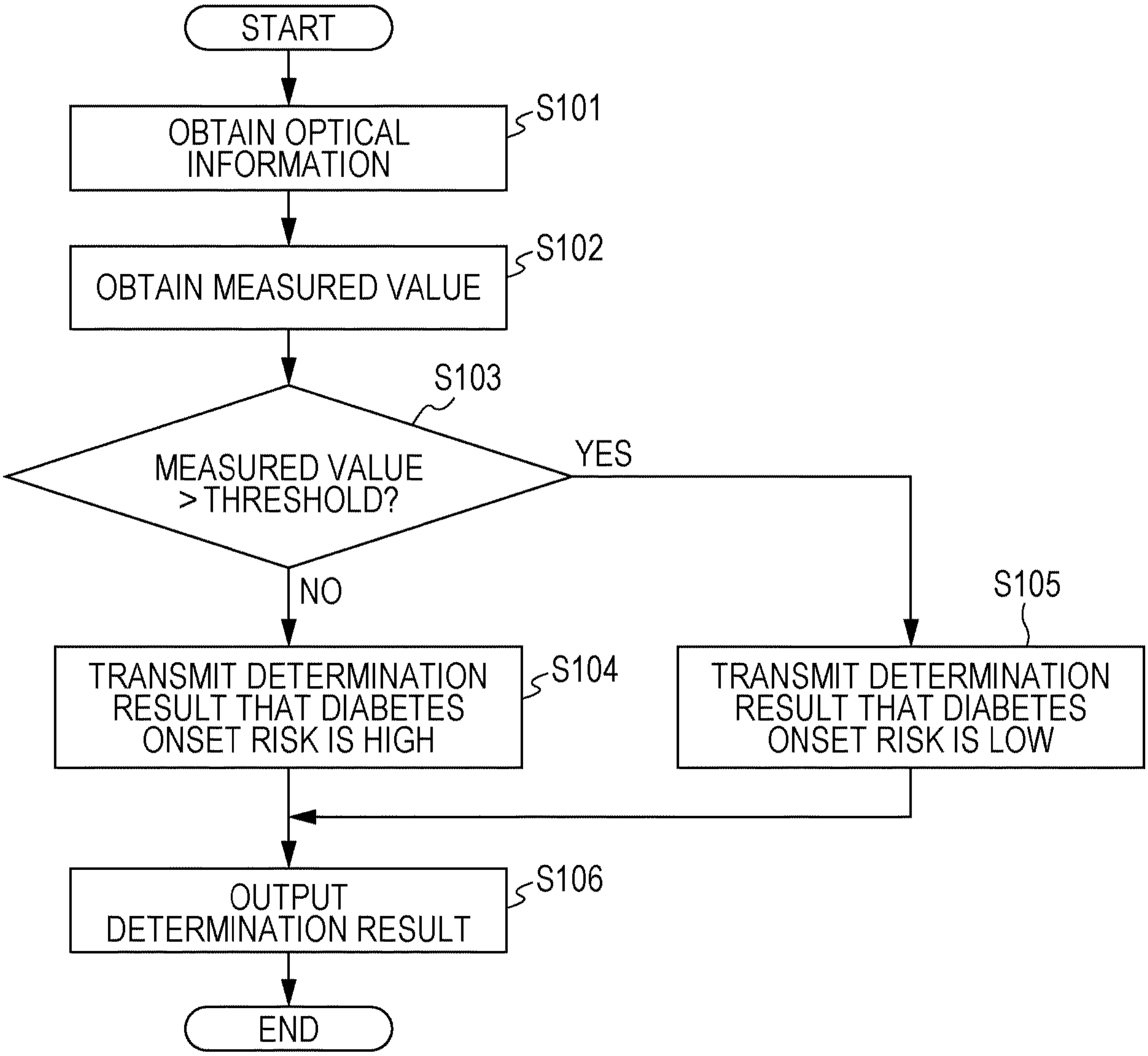
START
OBTAIN OPTICAL INFORMATION
S101
OBTAIN MEASURED VALUE
S102
S103
MEASURED VALUE > THRESHOLD?
YES
NO
S105
TRANSMIT DETERMINATION RESULT THAT DIABETES ONSET RISK IS HIGH
S104
TRANSMIT DETERMINATION RESULT THAT DIABETES ONSET RISK IS LOW
OUTPUT DETERMINATION RESULT
S106
END

FIG. 6

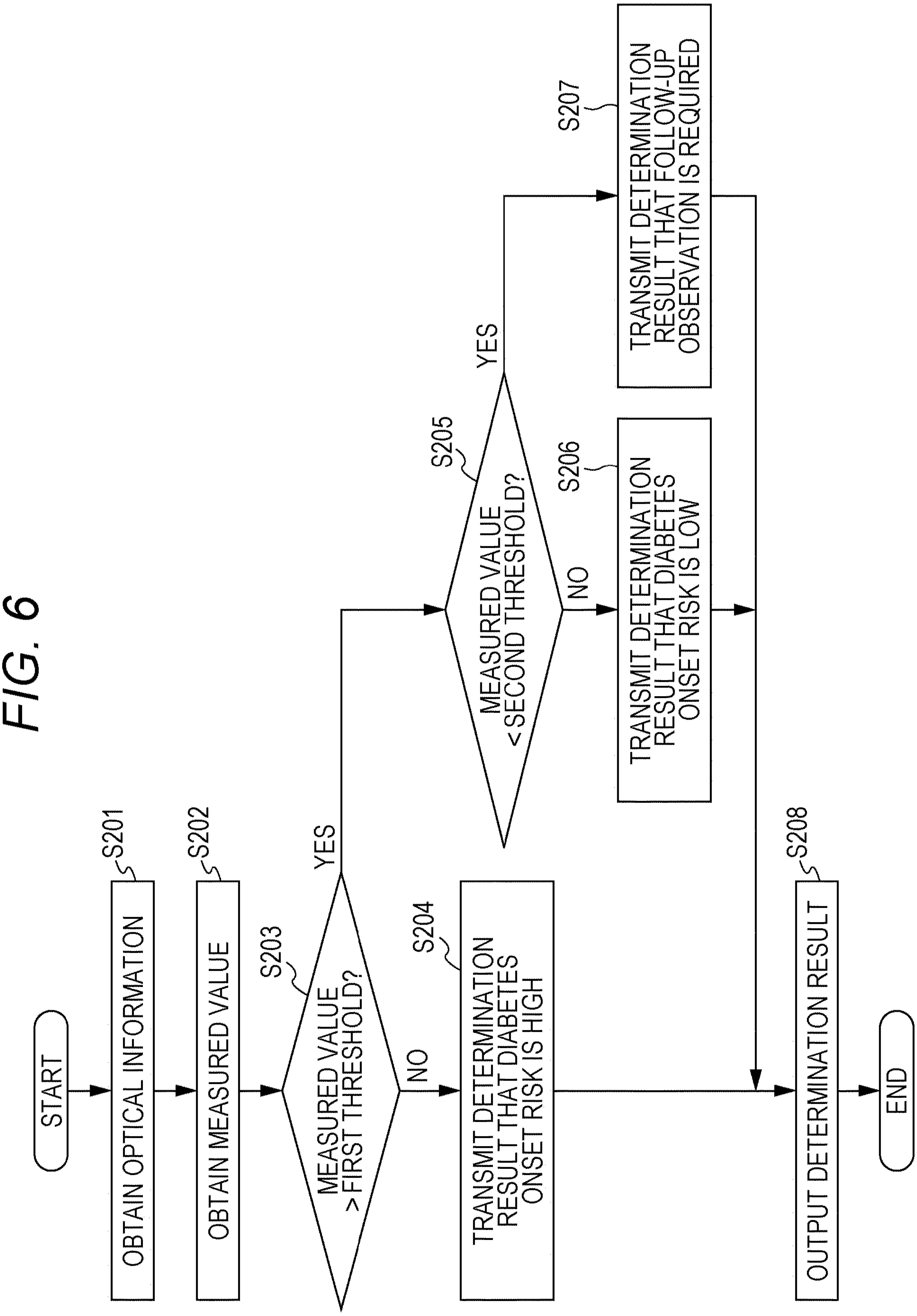
START
S201
OBTAIN OPTICAL INFORMATION
S202
OBTAIN MEASURED VALUE
S203
MEASURED VALUE >FIRST THRESHOLD?
YES
NO
S204
TRANSMIT DETERMINATION RESULT THAT DIABETES ONSET RISK IS HIGH
S205
MEASURED VALUE <SECOND THRESHOLD?
YES
NO
S206
TRANSMIT DETERMINATION RESULT THAT DIABETES ONSET RISK IS LOW
S207
TRANSMIT DETERMINATION RESULT THAT FOLLOW-UP OBSERVATION IS REQUIRED
S208
OUTPUT DETERMINATION RESULT
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20130143748 A1
- US 20170315112 A1


### Non-patent literature cited in the description


- **BLANCO-ROJO, R. et al.** HDL cholesterol efflux normalized to apoA-I is associated with future development of type 2 diabetes: from the CORDIOPREV trial. *Sci. Rep.,* 2017, vol. 7 (1), 12499
- **PITTO-BARRY A. ; BARRY N.P.E.** *Poly. Chem.,* 2014, vol. 5, 3291-3297